(19) Europäisches Patentamt
European Patent Office
Office européen des brevets

(11) Veröffentlichungsnummer: 0 222 191
B1

(12) EUROPÄISCHE PATENTSCHRIFT

(45) Veröffentlichungstag der Patentschrift: 30.01.91

(21) Anmeldenummer: 86114241.2

(22) Anmeldetag: 15.10.86

(51) Int. Cl.[5]: C 07 D 401/04, C 07 D 403/04, C 07 D 451/04, C 07 D 403/06, A 61 K 31/50

# (54) 4-Benzyl-1-(2H)-phthalazinon-Derivate.

(30) Priorität: 11.11.85 DE 3539873

(43) Veröffentlichungstag der Anmeldung: 20.05.87 Patentblatt 87/21

(45) Bekanntmachung des Hinweises auf die Patenterteilung: 30.01.91 Patentblatt 91/05

(84) Benannte Vertragsstaaten: AT BE CH DE ES FR GB GR IT LI LU NL SE

(56) Entgegenhaltungen:
EP-A-0 174 464
US-A-3 813 384

CHEMICAL ABSTRACTS, Band 96, Nr. 1, 4. Januar 1982, Columbus, Ohio, USA; SUZUKI, Y., OKADA, F., MIKAMI, T., GOTO, M., HASEGAWA, H., CHIBA, T.: "Teratology and reproduction studies of azelastine, a novel antiallergic agent, in rats and rabbits" Seite 40, Spalte 1, Zusammenfassung Nr. 466p

(73) Patentinhaber: ASTA Pharma AG
Weismüllerstrasse 45
D-6000 Frankfurt am Main 1 (DE)

(72) Erfinder: Engel, Jürgen, Dr.
Erlenweg 3
D-8755 Alzenau (DE)
Erfinder: Scheffler, Gerhard, Dr.
Frankenwaldstrasse 19
D-6450 Hanau 6 (DE)

(56) Entgegenhaltungen:
CHEMICAL ABSTRACTS, Band 92, Nr. 23, 9. Juni 1980, Columbus, Ohio, USA; TATSUMI, K., OU, T., YAMADA, H., YOSCHIMURA, H.: "Studies on metabolic fate of a new antiallergic agent, azelastine (4-(p-chlorobenzyl)-2- N-methylperhydroazepinyl-(4) -1-(2H)-phthalazinone hydrochloride)" Seite 21, Spalte 1, Zusammenfassung Nr. 191057f

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Courier Press, Leamington Spa, England.

**Beschreibung**

Gegenstand der deutschen Patentschrift 21 64 058 sind basisch substituierte 4-Benzyl-1-(2H)-phthalazinonDerivate der folgenden Formel

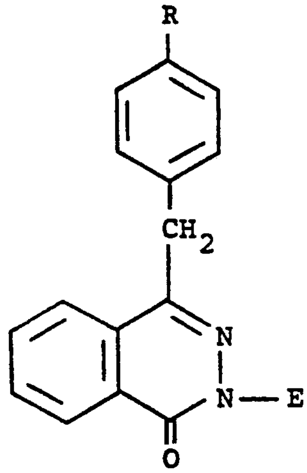

worin R ein Wasserstoff- oder Halogenatom, eine Trifluormethylgruppe oder eine niedere Alkyl- oder Alkoxygruppe und E einen 4-Perhydroazepinyl-, N-Methyl-4-perhydroazepinyl-, 3-Chinuclidyl-, 3-Tropanyl-, 3-Nortropanyl-, N-Methyl-3-pyrrolidinyl- oder N-Methyl-2-pyrrolidinyl-methyl-Rest bedeutet, sowie die physiologisch unbedenklichen Säureadditionssalze hiervon.

Diese Verbindungen besitzen eine Antihistamin-Wirkung. Die Erfindung betrifft die durch die Patentansprüche definierten Gegenstände.

Die erfindungsgemäßen Verbindungen sind antiallergisch, histaminolytisch und asthmaprophylaktisch wirksam, jedoch erheblich stärker und besser als die vorbekannten Verbindungen der deutschen Patentschrift 21 64 058. Darüberhinaus besitzen sie im Gegensatz zu dem bekannten Arzneimittelwirkstoff AZELASTIN (Verbindung gemäß Beispiel 5 der deutschen Patentschrift 21 64 058) keinen, beziehungsweise einen erheblich geringeren bitteren Geschmack, so daß sie ohne weiteres beispielsweise auch als Aerosol appliziert werden können. Weiterhin besitzen die erfindungsgemäßen Verbindungen auch eine analgetische Wirkung, insbesondere peripher-analgetische Wirkung. Diese analgetische Wirkung wird beispielsweise durch den Randall-Selito-Test an der Ratte (Entzündungsschmerz an der Ratte) nachgewiesen.

Der Rest $R_1$ befindet sich vorzugsweise in 4-Stellung 1 des Phenylringes; der Rest $R_2$ vorzugsweise in der 3-Stellung. Vorkommende $C_1$—$C_6$-Alkylgruppen, $C_1$—$C_6$-Alkoxygruppen oder Alkanoylgruppen (zum Beispiel $C_2$—$C_6$-Alkanoyloxygruppen) können gerade oder verzweigt sein, insbesondere bestehen diese Reste aus 1—4 beziehungsweise 2—4 C-Atomen.

Der Rest $R_3$ befindet sich in 5, 6, 7 und 8-Stellung des 1-(2H)-Phthalazinon-Restes, vorzugsweise in der 6- und/oder 7-Stellung.

Falls $R_5$ eine $C_1$—$C_6$-Alkylgruppe ist, die durch die angegebenen Reste substituiert ist, besteht $R_5$ vorzugsweise aus einem, zwei oder drei C-Atomen, wobei der Substituent sich dann insbesondere in der ω-Stellung befindet.

Besonders günstige Wirkungen besitzen zum Beispiel solche Verbindungen, wo die Reste $R_1$ bis $R_3$, A und $R_5$ die folgenden Bedeutungen haben: $R_2$ und $R_3$ = Wasserstoff; $R_1$ = Fluor, Chlor oder Brom, insbesondere in 4-Stellung, vorzugsweise Fluor in 4-Stellung; A = der Hexahydroazepin-4-yl-rest oder der Piperidyl-(4)-rest; $R_5$ = eine $C_1$—$C_6$-Alkylgruppe (vorzugsweise Methyl, Ethyl, Propyl, Isopropyl), die in ω-Stellung einen unsubstituierten oder substituierten Phenylrest enthält. Bei den Substituenten dieses Phenylrestes handelt es sich zum Beispiel um eine $C_1$—$C_4$-Alkylgruppe (insbesondere Methyl) oder um ein Halogen (zum Beispiel Cl, F) oder drei $C_1$—$C_4$-Alkoxygruppen (insbesondere Methoxygruppen) in 3,4- und 5-Stellung.

Falls der Phenylrest keinen Substituenten enthält, ist $R_1$ vorzugsweise Fluor in 4-Stellung, $R_2$ und $R_3$ Wasserstoff, A Piperidyl-(4)- oder vorzugsweise Hexahydroazepin-4-yl und $R_5$ Phenylmethyl, Phenylethyl, 3-Phenyl-propyl oder 2-Phenyl-propyl-(1).

Zu dem Verfahren a)

Das Verfahren kann ohne zusätzliches Lösungsmittel oder in einem geeigneten Lösungs- oder Dispergiermittel durchgeführt werden. Als Lösungs- oder Dispergiermittel kommen zum Beispiel in Betracht: Aromatische Kohlenwasserstoffe wie zum Beispiel Benzol, Mesitylen, Toluol, Xylol; Pyridin; niedere aliphatische Ketone wie zum Beispiel Aceton, Methyläthylketon; halogenierte Kohlenwasserstoffe wie zum Beispiel Chloroform, 1,2-Dichlorethan, Tetrachlorkohlenstoff, Chlorbenzol, Methylenchlorid; Ether

wie zum Beispiel Tetrahydrofuran, Dioxan, Diisopropylether; Sulfoxyde wie zum Beispiel Dimethylsulfoxid; tertiäre Säureamide wie zum Beispiel Dimethylformamid, Dimethylacetamid, Hexamethylphosphorsäuretriamid, Tetramethylharnstoff, N-Methylpyrrolidon; niedere Alkohole wie zum Beispiel Methanol, Äthanol, Isopropanol, Amylalkohol, Butanol, tert.-Butanol sowie Mischungen der genannten Mittel. Die Reaktion wird beispielsweise bei Temperaturen zwischen 20° bis 200°C, vorzugsweise 40 bis 160°C oder auch 50 bis 120°C durchgeführt. Wird ein Lösungs- beziehungsweise Dispersionsmittel verwendet, arbeitet man häufig bei der Rückflußtemperatur dieses Mittels. Die Reaktion läuft häufig bereits auch schon bei Raumtemperatur ab, beziehungsweise bei einer Temperatur zwischen 40 bis 120°C.

Die Umsetzung wird vorteilhaft in Gegenwart von säurebindenden Mitteln wie Alkalicarbonaten, Pottasche, Soda, Alkaliacetaten, Alkalihydroxyden oder tertiären Basen (Triethylamin, Pyridin) durchgeführt.

Falls X eine veresterte Hydroxygruppe bedeutet, dann handelt es sich hierbei um reaktionsfähige Ester. Ein reaktionsfähiger Ester ist dabei zum Beispiel derjenige einer starken organischen oder anorganischen Säure, wie vor allem einer Halogenwasserstoffsäure, zum Beispiel der Chlor-, Brom- oder Jodwasserstoffsäure, oder einer Sulfonsäure, wie einer Aryl- oder $C_1$—$C_6$-Alkylsulfonsäure, zum Beispiel von niederen Alkylbenzolsulfonsäuren (p-Toluolsulfonsäure). Als Lösungsmittel kommen insbesondere Mittel wie Dioxan/Wasser, Dimethylformamid/Wasser oder niedere gesättigte aliphatische Alkohole in Frage.

Nicht bekannte Ausgangsstoffe der Formel III können zum Beispiel analog Houben-Weyl, Methoden der Organischen Chemie, Band 5/3 (1962), Seite 503 ff., Band 6/2 (1963), Seite 475 ff. oder Band 9 (1955), Seite 426 erhalten werden.

Herstellung von Ausgangsstoffen der Formel II:

Die Ausgangsverbindung II, worin $R_1$ Fluor in 4-Stellung, $R_2$ Wasserstoff und A der Piperidyl-(4)-rest ist, wird beispielsweise wie folgt erhalten:

N-Benzoyl-N'-(N-methyl-piperidin-4-yl)-hydrazin

In 1500 ml Methanol werden unter Rühren 272 g (2 mol) Benzoesäurehydrazid eingetragen. Innerhalb einer Stunde werden dann bei einer Temperatur von 30°C 226 g (2 mol) 1-Methyl-4-piperidinon zugetropft. Eine leichte Kühlung ist erforderlich. Das Reaktionsgemisch wird eine halbe Stunde bei 30°C und dann noch 1 Stunde bei 60°C nachgerührt. Die Lösung wird auf 0°C abgekühlt und spatelweise innerhalb von 2 Stunden 68 g (1,8 mol) $NaBH_4$ eingetragen (starkes Schäumen). Gegen Ende der Zugabe wird auf Raumtemperatur erwärmt. Das Lösungsmittel wird am Rotationsverdampfer abgedampft und zum Rückstand werden 900 g Eis und 1000 ml Dichlormethan gegeben und gerührt. Die organische phase wird am Scheidetrichter abgetrennt und die wäßrige Phase noch 2mal mit je 300 ml Dichlormethan ausgeschüttelt. Die gesammelten organischen Fraktionen werden über $MgSO_4$ getrocknet, das Dichlormethan abgedampft und der Rückstand in 1 Liter Isopropanol unter Zusatz von 10 g Aktiv-Kohle umkristallisiert. Das Reaktionsprodukt wird im Trockenschrank bei 50°C unter Vakuum getrocknet. (F. 149—150°C).

4-Hydrazino-N-methylpiperidin × 2 HCl

Zu 400 ml Wasser werden 700 ml 37 %ige HCl (8,4 mol) gegeben, unter Rühren 233 g (1 mol) N-Benzoyl-N'-(N-methyl-piperidin-4-yl)-hydrazin eingetragen und das Reaktionsgemisch 2 Stunden unter Rückfluß gekocht. Die freiwerdende Benzoesäure kristallisiert aus. Es wird im Eisbad abgekühlt und abgesaugt. Das Filtrat wird am Rotationsverdampfer eingedampft. Um eventuell vorhandenes Wasser zu entfernen, werden 250 ml Methanol zugegeben und wieder abgedampft. Durch Verrühren der schmierigen Substanz in 300 ml Methanol erhält man ein kristallines Produkt, welches abgesaugt, mit Methanol gewaschen und unter Vakuum bei 50°C getrocknet wird. (F. 173°C).

2-(4-Fluorphenacetyl)-benzoesäure

Es werden 410 ml 29 %ige NaOH vorgelegt und bei einer Ölbadtemperatur von 70°C unter Rühren 250 g (1,04 mol) 4-Fluorbenzylidenphthalid eingetragen. Gegen Ende der Zugabe wird die Ölbadtemperatur auf 110°C erhöht. Während der Reaktion entsteht ein rotes Öl. Nach 3 Stunden wird abgekühlt, mit 500 ml Eiswasser versetzt und 340 ml konzentrierte HCl zugetropft. Hierbei scheiden sich rosa gefärbte Kristalle ab. Diese werden abgesaugt und mit Eiswasser chloridfrei gewaschen. Nach dem Trocknen bei 50°C wird aus 900 ml Toluol umkristallisiert. (F. 147-150°C).

4-(4-Fluorbenzyl)-2-(N-methyl-piperidin-4-yl)-1-(2H)-phthalazinon

112,3 g KOH 85 % (1,7 mol) werden in 1890 ml Methanol unter Rühren gelöst. 214 g (0,945 mol) 4-Hydrazino-N-methylpiperidin × 2 HCl werden eingetragen und die Base freigesetzt. Das entstehende KCl kristallisiert aus. Nach der Zugabe von 244 g (0,945 mol) 2-(4-Fluorphenacetyl)benzoesäure wird 4 Stunden unter Rückfluß gerührt (im Falle anderer Hydrazin-Verbindungen und/oder Phenacetylbenzoesäuren dauert diese Umsetzung gegebenenfalls länger, beispielsweise bis zu 20 Stunden). Nach dem Abkühlen wird das KCl abgetrennt und die Lösung am Rotationsverdampfer eingeengt. Es werden 1000 ml Eiswasser und 928

ml NaOH (2 molar) zugegeben und gerührt. Die N-Methylverbindung fällt aus und wird abgesaugt, mit Eiswasser gewaschen und im Trockenschrank bei 50°C unter Vakuum getrocknet (F. 140—143°C).

4-(4-Fluorbenzyl)-2-(N-carbethoxy-piperidin-4-yl)-1-(2H)-phthalazinon

366,5 g (1,043 mol) 4-(4-Fluorbenzyl)-2-(N-methylpiperidin-4-yl)-1-(2H)-phthalazinon werden in 1100 ml über $K_2CO_3$ getrocknetem Toluol gelöst. Eventuell noch vorhandenes Wasser wird über einen Wasserabscheider azeotrop abdestilliert. 390 g (3,59 mol) Chlorameisensäureethylester werden in 1½ Stunden zugetropft und 4 Stunden unter Rückfluß gerührt. Es fällt ein Niederschlag aus, der abgesaugt wird. Das Filtrat wird am Rotationsverdampfer eingedampft, der Rückstand in 400 ml Ether aufgeschlämmt, kurz gerührt und abgesaugt. Nach dem Waschen mit Ether wird die Substanz im Trockenschrank getrocknet. (F. 151—152°C).

4-(4-Fluorbenzyl)-2-(piperidin-4-yl)-1-(2H)-phthalazinon

181,5 g 48 %ige HBr (1,077 mol) werden vorgelegt und unter Rühren 110,2 g (0,269 mol) des vorstehend beschriebenen N-Carbethoxy-piperidin-Derivats eingetragen. Es wird Eisessig bis zur klaren Lösung zugetropft und bis zu einer Ölbadtemperatur von 100°C aufgeheizt. Nach 9 Stunden wird das Lösungsmittel abgedampft. Zu der zurückbleibenden zähen Masse werden 860 ml Wasser gegeben und gerührt, woraufhin weiße Substanz auskristallisiert. Bei der Zugabe von 138 ml konzentriertem Ammoniak fällt das Reaktionsprodukt als Base aus. Diese wird abgesaugt, mit Wasser gewaschen und im Trockenschrank bei 50°C getrocknet. (F. 157—159°C).

Ausgangs-Hydrazino-Verbindungen mit dem Rest A, worin $R_5$ die Methylgruppe ist, können zum Beispiel auch wie folgt erhalten werden:

In 120 ml Toluol werden 41,0 g (0,295 mol) Tropinon (N-methyl-8-azabicyclo[3.2.1][octan-3-on) und dann 22,8 g (0,31 mol) Acetylhydrazin eingetragen. Es wird 3 Stunden unter Rückfluß gekocht und dabei das entstehende Wasser azeotrop abdestilliert. Über Nacht kristallisiert das entstandene Produkt aus. Das restliche Lösungsmittel wird abgedampft und das erhaltene N-acetyl-N'-[3-(N-methyl-8-azabicyclo[3.2.1]octan]hydrazon aus 400 ml Essigsäureethylester umkristallisiert (F. 155—157°C).

58 g (0,297 mol) dieser Verbindung, 440 ml Eisessig und 5,8 g $PtO_2$ (81,05 %) werden nun in einen Schüttel-autoklaven gegeben und ein Wasserstoffdruck von ca. 5,7 bar aufgegeben. Es wird bei Raumtemperatur hydriert. Nach ca. 4 Stunden ist die $H_2$-Aufnahme beend et. Der Katalysator wird abfiltriert und das Filtrat am Rotationsverdampfer eingeengt. Die N-Acetyl-hydrazin-Verbindung bleibt als öliger Rückstand zurück.

Zu 170,5 g des so erhaltenen öligen Rückstandes werden 1200 ml 22 %ige HCl gegeben. Das Gemisch wird in Stickstoffatmosphäre 8 Stunden unter Rückfluß erhitzt. Die entstandene Lösung wird am Rotationsverdampfer eingedampft, wobei eine kristalline Substanz zurückbleibt. Zur Entfernung von eventuell noch vorhandenem Wasser wird 2mal mit je 150 ml Methanol versetzt und wieder abgedampft. Der Rückstand wird im Trockenschrank bei 50°C unter Vakuum getrocknet. Das so erhaltene 3-Hydrazino-N-methyl-8-azabicyclo[3.2.1]octan × 2 HCl schmilzt bei 250°C unter Zersetzung.

Andere Ausgangsverbindung der Formel II können in analoger Weise erhalten werden. Die entsprechenden 2-Phenacetyl-benzoesäuren enthalten im Phenacetylrest die Reste $R_1$ und $R_2$ und im Benzoesäureteil den Rest $R_3$. Man stellt also zweckmäßig zuerst die entsprechende Verbindung II her, wo $R_5$ des Restes A eine Methylgruppe ist und spaltet diese über die Carbethoxy-Verbindung ab. Die zugrunde liegenden Verbindungen II, worin $R_5$ die Methylgruppe ist, können auch gemäß beziehungsweise analog der deutschen Offenlegungsschrift 21 64 058 hergestellt werden.

Zu dem Verfahren b)

Als reaktionsfähige Derivate der Carbonsäure der allgemeinen Formel IV kommen insbesondere die Säurehalogenide (-chloride, -bromide, -jodide), -ester (insbesondere mit $C_1$—$C_6$-Alkanolen; oder auch innere Ester mit der enolisierten Ketogruppe (beispielsweise p-Chlor-benzyliden-phthalid) und -anhydride in Frage. Die Verbindungen der Formel IV sowie der entsprechenden Säurehalogenide und Ester mit

$C_1$—$C_6$-Alkanolen können auch in der tautomeren cyclischen Form vorliegen. Diese cyclische Form wird durch folgende Formel ausgedrückt:

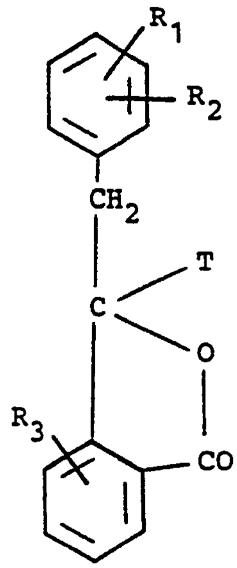

Hierbei bedeutet T die Hydroxygruppe, Halogen oder $C_1$—$C_6$-Alkoxy.

Die Umsetzung wird in An- oder Abwesenheit der üblichen Lösungs- und Hilfsmittel bei Temperaturen zwischen 40 und 200°C und in weitem pH-Bereich vom Sauren bis zum Alkalischen durchgeführt.

Als Lösungsmittel eignen sich zum Beispiel Wasser, aromatische Kohlenwasserstoffe wie zum Beispiel Benzol, Mesitylen, Toluol, Xylol; halogenierte Kohlenwasserstoffe wie zum Beispiel Chloroform, 1,2-Dichlorethan, Tetrachlorkohlenstoff, Chlorbenzol, Methylenchlorid; Ether wie zum Beispiel Tetrahydrofuran, Dioxan, Diisopropylether; Sulfoxyde wie zum Beispiel Dimethylsulfoxid; tertiäre Säureamide wie zum Beispiel Dimethylformamid, Dimethylacetamid, Hexamethylphosphorsäuretriamid, Tetramethylharnstoff, N-Methylpyrrolidon; niedere Alkohole wie zum Beispiel Methanol, Äthanol, Isopropanol, Amylalkohol, Butanol, tert.-Butanol und Mischungen der genannten Mittel sowie auch tertiäre Amine, zum Beispiel Pyridin. Als Hilfsmittel können in Frage kommen Basen, Säuren und für diese Reaktionen übliche Kondensationsmittel.

Die Verbindung V kann auch in Form eines N-Acylderivats eingesetzt werden, das dann zuerst hydrolisiert wird und ohne weitere Reinigung oder Isolierung in demselben Reaktionsmedium sofort weiter mit der Verbindung IV umgesetzt wird.

Für die Umsetzung von solchen Benzyl-phthalazinon-Derivaten, die erhalten werden, wenn Z der Formel V Wasserstoff ist, mit einer Verbindung Y—A, kommen ebenfalls die oben angegebenen Lösungsmittel sowie der oben angegebene Temperaturbereich in Frage.

Insbesondere kommen als Lösungsmittel tertiäre Säureamide (zum Beispiel Dimethylformamid), aromatische Kohlenwasserstoffe (zum Beispiel Toluol), niedere Alkohole oder auch Wasser in Frage, wobei häufig in Gegenwart basischer Stoffe (zum Beispiel Alkalihydroxiden beziehungsweise Alkalialkoholaten) gearbeitet wird. Vorzugsweise wird bei Temperaturen zwischen 50—200°C, insbesondere 80—150°C gearbeitet.

Falls Y der Formel VI ein Halogenatom bedeutet, handelt es sich um Chlor, Brom oder Jod. Falls Y der Formel VI eine Sulfonsäureestergruppe darstellt, handelt es sich beispielsweise um einen $C_1$—$C_6$-Alkylsulfonsäurerest (zum Beispiel $CH_3$—$SO_2$—O—) oder einen Arylsulfonsäurerest, wie zum Beispiel den Rest einer $C_1$—$C_4$-Alkylbenzolsulfonsäure (zum Beispiel p-Toluolsulfonyloxyrest). Die Benzyl-phthalazinon-Ausgangsverbindung (Verbindung der Formel I, bei der sich an dem Säureamid-Stickstoffatom anstelle des Restes A ein Wasserstoffatom befindet) wird beispielsweise auch in Form ihres Alkalisalzes (Na, K) eingesetzt. Derartige Alkalisalze können beispielsweise in üblicher Weise aus dem entsprechenden Phthalazinon und dem Alkalimetall in alkoholischer Lösung (zum Beispiel Ethanol) oder einem anderen hierfür üblichen Mittel zwischen 40 und 100°C erhalten werden. Falls bei der Ausgangsverbindung der Formel V der Rest Z Wasserstoff ist, entstehen bei der anschließenden Umsetzung des entstandenen 4-Benzylphthalazinons mit der Verbindung VI, worin A entweder den Rest

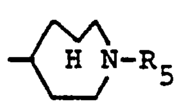

oder den Rest

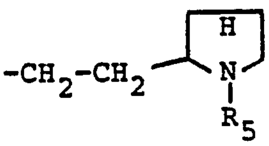

bedeutet, als Endprodukte jeweils Gemische aus Verbindungen der Formel I, worin A den Hexahydroazepin-Rest

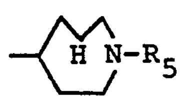

und Verbindungen der Formel I, worin A den 2-Pyrrolidinethylrest

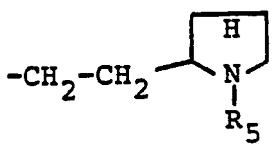

darstellt (Cyclammonium-Umlagerung). Die Isolierung der entsprechenden reinen Verbindungen kann beispielsweise in üblicher Weise durch fraktionierte Kristallisation erfolgen.

Herstellung von Ausgangsverbindungen der Formel V:
Solche Ausgangsstoffe können beispielsweise wie folgt erhalten werden:

Umsetzung der entsprechenden cyclischen Ketonen der Formel 0 = A' mit Acetylhydrazin (zum Beispiel in einem aromatischen Kohlenwasserstoff (Toluol) zwischen 50—150°C) oder Benzoylhydrazin (zum Beispiel in einem aliphatischen Alkohol zwischen 20—100°C) zu den entsprechenden Hydrazonen, anschließende Reduktion derselben durch katalytische Hydrierung (zum Beispiel in Eisessig in Gegenwart von $PtO_2$ bei 5—6 bar) oder Reduktion mit einem komplexen Metallhydrid ($NaBH_4$) in einem inerten Mittel (niedere Alkohole wie Methanol oder Dioxan) und anschließende Abspaltung der Acylgruppe durch Hydrolyse mit verdünnter Salzsäure (20—37 %ig). Bei den Ketonen der Formel 0 = A' bedeutet A' den Rest A, wobei über das tertiäre Kohlenstoffatom (über welches A mit dem Phthalazinon-Rest verbunden ist) anstelle einer Wasserstoffbindung die zweite Bindung mit dem Keto-Sauerstoff erfolgt.

Eine andere Möglichkeit ist beispielsweise die Umsetzung eines Acylhydrazins (zum Beispiel Benzoylhydrazin) mit einer Verbindung der Formel VI in einem inerten Mittel (zum Beispiel Dimethylformamid) zwischen 50 und 180°C. in Gegenwart eines säurebindenden Mittels (tertiäre Amine).

Die verschiedenen Reste $R_5$ können beispielsweise vor der Umsetzung mit dem Acylhydrazin aus den cyclischen Ketonen, bei denen $R_5$ Wasserstoff ist, durch Umsetzung mit einer Verbindung $R_5X$ (X = Cl, Br, J oder Alkyl- oder Arylsulfonsäurerest) in einem inerten Lösungsmittel, gegebenenfalls in Gegenwart einer basischen Verbindung bei Temperaturen zwischen 20—200°C erhalten werden.

Ausgangsstoffe der Formel IV können beispielsweise in bekannter Weise durch übliche Perkin-Synthese aus einem den Rest $R_3$ enthaltenden Phthalsäure-anhydrid und der durch die Reste $R_1$ und $R_2$ substituierten Phenylessigsäure erhalten werden.

Bei der Einführung einer $C_2MC_6$-Alkanoylgruppe durch Acylierung handelt es sich um die entsprechende Acylierung von Verbindungen der Formel I, bei denen die Reste $R_1$, $R_2$ Hydroxygruppen, Aminogruppen oder $C_1$—$C_6$-Alkylaminogruppen darstellen. Die Acylierung erfolgt mittels einer $C_2$-$C_6$-Alkancarbonsäure, wobei diese vorzugsweise aktiviert ist. Falls für die Acylierung eine solche aktivierte Säure verwendet wird, handelt es sich vorzugsweise um Verbindungen der Formel $C_2$-$C_6$-Alkanoyl-W, worin W Halogen (zum Beispiel Chlor oder Brom) ist, aber auch zum Beispiel eine Gruppe der Formel —OR',' -SR' oder eine Gruppe der Formel —$OSO_3H$ oder —OCO—R'' bedeutet und R' einen $C_1$—$C_6$-Alkylrest oder auch einen Phenylrest, einen durch Nitrogruppen, $C_1$—$C_4$-Alkoxygruppen, $C_1$—$C_4$-Alkylgruppen oder Halogenatome (Chlor, Fluor, Brom) substituierten Phenylrest, einen Cyanmethylrest oder einen Carboxymethylrest und R'' einen geraden oder verzweigten $C_1$—$C_5$-Alkylrest darstellt. Falls W ein Halogenatom bedeutet handelt es sich vorzugsweise um Chlor, Brom oder Jod; falls R' beziehungsweise R'' Alkylreste, Alkylthioreste oder Alkoxyreste bedeuten, dann sind diese vorzugsweise niedermolekular und bestehen aus 1—4 Kohlenstoffatomen. Häufig, insbesondere wenn W ein Halogenatom oder die Gruppe —OCOR'' bedeutet, ist die Gegenwart eines säurebindenen Stoffes wie Alkalihydroxiden, Alkalicarbonaten, Alkalihydrogencarbonaten, Alkaliacetaten, Erdalkalicarbonaten, tertiären Aminen (Trialkylamine wie Triethylamin, Pyridin) oder auch Alkalihydriden und ähnlichen zweckmäßig. Dabei kann das säurebindende Agens auch gleichzeitig allein oder im Gemisch mit anderen

üblichen Mitteln als Lösungsmittel benutzt werden (zum Beispiel Pyridin).

Falls die freie Säure verwendet wird, so ist deren Aktivierung durch die Gegenwart von Kondensationsmitteln wie Dicyclohexylcarbodiimid, Tetraäthylpyrophosphit, 5-(3'-Sulfonphenyl)-äthylisooxazol, Schwefligsäure-bis-alkylamiden (zum Beispiel $SO[N(CH_3)_2]_2$, N,N'-Carbonyldiimidazol und so weiter erforderlich (Organic Reactions, Vol. 12, 1962, Seiten 205 und 239).

Bei der Einführung einer $C_1$—$C_6$-Alkylgruppe beziehungsweise der Benzylgruppe handelt es sich um die Alkylierung von Verbindungen der Formel I, bei denen die Reste $R_1$, $R_2$ Hydroxygruppen, Aminogruppen oder Alkanoylaminogruppen darstellen. Diese Alkylierung (beziehungsweise Benzylierung) erfolgt beispielsweise durch Umsetzung mit Verbindungen der Formel $C_1$—$C_6$-Alkyl-Hal, Benzyl-Hal, $ArSO_2O$—$C_1$—$C_6$-Alkyl und $SO_2(O$—$C_1$—$C_6$-Alkyl$)_2$, wobei Hal ein Halogenatom (insbesondere Chlor, Brom oder Jod) und Ar ein aromatischer Rest (zum Beispiel ein gegegebenenfalls durch einen oder mehrere niedere Alkylreste substituierter Phenyl- oder Naphthylrest sein kann). Beispiele sind p-Toluolsulfonsäure-$C_1$—$C_6$-alkylester, $C_1$—$C_6$-Dialkylsulfate, $C_1$—$C_6$-Alkylhalogenide und ähnliche. Die Alkylierungsreaktion wird gegebenenfalls zweckmäßig in Gegenwart von üblichen säurebindenden Mitteln durchgeführt. Als säurebindende Mittel kommen beispielsweise die gleichen in Frage wie für die Acylierung.

Die Acylierung beziehungsweise die Alkylierung wird zum Beispiel bei Temperaturen zwischen −20 und 220°C, vorzugsweise 0 und 150°C, insbesondere 20°C und 80°C in inerten Lösungsmitteln oder Suspensionsmitteln durchgeführt. Als Lösungs- oder Dispergiermittel kommen beispielsweise in Betracht: aromatische Kohlenwasserstoffe wie zum Beispiel Benzol, Toluol, Xylol; aliphatische Ketone wie zum Beispiel Aceton, Methylethylketon; halogenierte Kohlenwasserstoffe, wie zum Beispiel Chloroform, Tetrachlorkohlenstoff, Chlorbenzol, Methylenchlorid; aliphatische Ether, wie zum Beispiel Butylether; cyclische Ether, wie zum Beispiel Tetrahydrofuran, Dioxan; Sulfoxide, wie zum Beispiel Dimethylsulfoxid; tertäre Säureamide, wie zum Beispiel Dimethylformamid, N-Methylpyrrolidon, Hexamethylphosphorsäuretriamid; aliphatische Alkohole wie Methanol, Ethanol, Isopropanol, Amylalkohol, tert.-Butanol, cycloaliphatische Kohlenwasserstoffe, wie Cyclohexan und ähnliche. Auch wässrige Mischungen der genannten Lösungsmittel können verwendet werden. Häufig arbeitet man bei der Rückflußtemperatur der verwendeten Lösungs- beziehungsweise Dispergiermittel. Häufig werden die Alkylierungsreaktionskomponenten im Überschuß eingesetzt. Die Alkylierung kann auch in Gegenwart von Tetraalkylammoniumsalzen (insbesondere der Halogenide) in Kombination mit Alkalihydroxiden bei Temperaturen zwischen 0—100°C, vorzugsweise 20—80°C in einem aprotischen Lösungsmittel oder auch in Chloroform oder Methylenchlorid vorgenommen werden. Als aprotische Lösungsmittel kommen insbesondere in Betracht: tertiäre Amide (Dimethylformamid, N-Methyl-pyrrolidon, Hexamethylphosphorsäuretriamid), Dimethylsufoxid, Acetonitril, Dimethyloxyethan, Aceton, Tetrahydrofuran. Bei der Acylierung und Alkylierung kann man auch so vorgehen, daß man erst von der umzusetzenden Verbindung eine Alkaliverbindung herstellt, indem man sie in einem inerten Lösungsmittel wie Dioxan, Dimethylformamid, Benzol oder Toluol mit einem Alkalimetall, Alkalihydriden oder Alkaliamiden (insbesondere Natrium oder Natriumverbindungen) oder Butyllithium bei Temperaturen zwischen 0 und 150°C umsetzt und zum Beispiel dann das acylierende beziehungsweise alkylierende Agens zufügt.

Anstelle der angeführten Alkylierungs- und Acylierungsmittel können auch andere in der Chemie gebräuchliche chemisch-äquivalente Mittel verwendet werden (siehe zum Beispiel auch L. F. und Mary Fieser "Reagents for Organic Synthesis", John Wiley and Sons, Inc., New York, 1967, Vol. 1, Seiten 1303—4 und Vol. 2, Seite 471).

Die Acylgruppen in den Verbindungen der Formel I können solvolytisch wieder abgespalten werden, wodurch die entsprechenden Verbindungen der Formel I erhalten werden, die freie Hydroxygruppen, Aminogruppen oder $C_1$—$C_6$-Alkylaminogruppen haben. Diese solvolytische Abspaltung erfolgt beispielsweise durch Verseifung mit verdünnten Säuren oder mittels basischer Substanzen (Pottasche, Soda, wässrige Alkalilösungen, $NH_3$) bei Temperaturen zwischen 10 und 150°C insbesondere 20—100°C.

Je nach den Verfahrensbedingungen und Ausgangsstoffen erhält man die Endstoffe der Formel I in freier Form oder in Form ihrer Salze. Die Salze der Endstoffe können in an sich bekannter Weise, beispielsweise mit Alkali oder Ionenaustauschern, wieder in die Basen übergeführt werden. Von den letzteren lassen sich durch Umsetzung mit organischen oder anorganischen Säuren, insbesondere solchen, die zur Bildung von therapeutisch verwendbaren Salzen geeignet sind, Salze gewinnen.

Die erfindungsgemäßen Verbindungen der Formel I enthalten teilweise ein asymmetrisches Kohlenstoffatom (zum Beispiel C-Atom des 7-Ringes, welches mit dem Säureamid-Stickstoffatom des Phthalazinons verbunden ist) und werden in der Regel als Racemate erhalten. Solche Racemate können in an sich bekannter Weise beispielsweise durch fraktionierte Kristallisation der Salze von racemischen Verbindungen I mit optisch aktiven Säuren oder auch durch chromatographische Racemattrennung (siehe beispielsweise Angewandte Chemie 92/1 (1980) Seite 14) in die optisch aktiven Isomeren gespalten werden. Es ist aber auch möglich von vornherein eine optisch aktive Ausgangssubstanz einzusetzen wobei dann als Endprodukte eine entsprechende optisch aktive Form erhalten wird. Bei weiteren asymmetrischen C-Atomen erhält man diastereomere Gemische. Trennung kann nach den hierfür bekannten Methoden erfolgen.

Die vorliegende Erfindung umfasst also die Racemate und diastereomeren Formen sowie die entsprechenden optisch aktiven rechts- und linksdrehenden Formen.

Die erfindungsgemäßen Verbindungen sind zur Herstellung pharmazeutischer Zusammensetzungen

beziehungsweise Zubereitungen geeignet. Die pharmazeutischen Zusammensetzungen beziehungsweise Arzneimittel enthalten als Wirkstoff einen oder mehrere der erfindungsgemäßen Verbindungen, gegebenenfalls in Mischung mit anderen pharmakologisch beziehungsweise pharmazeutisch wirksamen Stoffen. Die Herstellung der Arzneimittel kann unter Verwendung der bekannten und üblichen pharmazeutischen Trägermittel und Hilfsstoffe erfolgen.

Die erfindungsgemäßen Verbindungen zeigen im allergischen (Ovalbumin-)Asthma am wachen Meerschweinchen eine gute asthmaprophylaktische und antiallergische Wirkung. Beispielsweise wird bei obengenannter Versuchsmethode bei einer Dosis von 0,5 mg/kg Körpergewicht am Meerschweinchen eine 50 %ige Schutzwirkung gegenüber dem allergisch induzierten Asthmaanfall erhalten.

Die niedrigste, bereits wirksame Dosis in dem oben angegebenen Tierversuch ist beispielsweise 0,1 mg/kg oral.

Als allgemeiner Dosisbereich für die Wirkung (Tierversuch wie oben) kommt beispielsweise in Frage: 0,01—3 mg/kg oral, insbesondere 0,1—1 mg/kg.

Die Wirkungsrichtung der erfindungsgemäßen Verbindungen ist mit der Wirkung des bekannten Wirkstoffs Dinatrium-Cromoglicinsäure vergleichbar, jedoch bestehen hierzu insbesondere folgende Unterschiede: gute perorale Wirkung; Leukotrien $C_4$-Antagonismus.

Indikationen, für die die erfindungsgemäßen Verbindungen in Betracht kommen können: Prophylaxe des Asthma bronchiale.

Kontraindikationen: keine.

Die pharmazeutischen Zubereitungen enthalten im allgemeinen zwischen 0,5 bis 30, vorzugsweise 1 bis 10, insbesondere 1—5 mg der erfindungsgemäßen aktiven Komponenten.

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen. Bevorzugte Anwendungsformen sind Tabletten, die zwischen 0,5 und 10 mg oder Lösungen, die zwischen 0,1 bis 5 % an aktiver Substanz enthalten.

Die Einzeldosis der erfindungsgemäßen aktiven Komponenten kann beispielsweise liegen

a) bei oralen Arzneiformen zwischen 0,5—10 mg, vorzugsweise 1—3 mg;

b) bei parenteralen Arzneiformen (zum Beispiel intravenös, intramuskulär) zwischen 0,05—3 mg, vorzugsweise 0,1—1 mg;

c) bei Arzneiformen zur Inhalation (Lösungen oder Aerosole) zwischen 0,1—2 mg, vorzugsweise 0,5—1 mg;

d) bei Arzneiformen zur rektalen oder vaginalen Applikation zwischen 0,1—10 mg, vorzugsweise 0,5—5 mg;

e) bei Arzneiformen zur lokalen Applikation auf die Haut und Schleimhäute (zum Beispiel in Form von Lösungen, Lotionen, Emulsionen, Salben und so weiter) zwischen 1—10 mg, vorzugsweise 0,5—5 mg.

Die Dosen sind jeweils bezogen auf die freie Base

Beispielsweise können 3 mal täglich 1 bis 3 Tabletten mit einem Gehalt von 0,5—5 mg wirksamer Substanz oder zum Beispiel bei intravenöser Injektion 1 bis 3 mal täglich eine Ampulle von 1 bis 5 ml Inhalt mit 1 bis 5 mg Substanz empfohlen werden. Bei oraler Verabreichung ist die minimale tägliche Dosis beispielsweise 0,5 mg; die maximale tägliche Dosis bei oraler Verabreichung soll nicht über 50 mg liegen.

Für die Behandlung von Hunden und Katzen liegt die orale Einzeldosis im allgemeinen zwischen ungefähr 0,03 und 5 mg/kg Körpergewicht; die parenterale Dosis ungefähr zwischen 0,01 und 3 mg/kg Körpergewicht.

Für die Behandlung von Pferden und Vieh liegt die orale Einzeldosis im allgemeinen zwischen ungefähr 0,03 und 5 mg/kg; die parenterale Einzeldosis ungefähr zwischen 0,01 und 3 mg/kg Körpergewicht.

Die akute Toxizität der erfindungsgemäßen Verbindungen an der Maus (ausgedrückt durch die LD 50 mg/kg; Methode nach Miller und Tainter: Proc. Soc. Exper. Biol. a. Med. *57* (1944) 261) liegt beispielsweise bei oraler Applikation zwischen 500 und 1000 mg/kg.

Die Arzneimittel können in der Humanmedizin, der Veterinärmedizin sowie in der Landwirtschaft allein oder im Gemisch mit anderen pharmakologisch aktiven Stoffen verwendet werden.

Zur Herstellung von entsprechenden Arzneimitteln wird mindestens eine Verbindung der Formel I mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht. Dies erfolgt zum Beispiel dadurch, daß man Verbindungen der Formel I, worin die Reste $R_1$, $R_2$, $R_3$, $R_5$ und A die in Anspruch 1 angegebene Bedeutung haben, beziehungsweise deren Salze mit physiologisch unbedenklichen Säuren zusammen mit üblichen Träger- und/oder Verdünnungs- beziehungsweise Hilfsstoffen bei Temperaturen zwischen 20 und 100°C vermischt beziehungsweise homogenisiert, die so erhaltene Mischung zur Herstellung von Zubereitungen, die in der Dosierungseinheit 0,1 bis 30 mg Wirkstoff der Formel I enthalten, in Hohlzellen entsprechender Größe ausgießt oder in Kapseln entsprechender Größe abfüllt oder granuliert und dann gegebenenfalls unter Zusatz von weiteren üblichen Hilfsstoffen zu Tabletten verpreßt. Zum Beispiel dadurch, daß man Verbindungen der Formel I, worin die Reste $R_1$, $R_2$, $R_3$, $R_5$ und A die in Anspruch 1 angegebene Bedeutung

haben, beziehungsweise deren Salze mit physiologisch unbedenklichen Säuren mit einem oder mehreren der folgenden Stoffe: Stärke, Cellulose, Lactose, Formalin-Casein, modifizierte Stärke, Magnesiumstearat, Calciumhydrogenphosphat, hochdisperse Kieselsäure, Talkum vermischt, die erhaltene Mischung, gegebenenfalls mit einer wässrigen Lösung, die als Bestandteil mindestens Gelatine, Stärke, Polyvinylpyrrolidon, Vinylpyrrolidon-Vinylacetat-Copolymerisat und/oder Polyoxyethylensorbitanmonooleat enthält, granuliert das Granulat gegebenenfalls mit einem oder mehreren der obengenannten Hilfsstoffe homogenisiert, und diese Mischung zu Tabletten verpresst oder in Kapseln abfüllt, wobei solche Tabletten oder Kapseln in der Dosierungseinheit jeweils 0,1 bis 30 mg Wirkstoff der Formel I enthalten; oder daß man Verbindungen der Formel I oder deren Salze mit physiologisch unbedenklichen Säuren nach Zusatz von Sojalecithin bei Temperaturen zwischen 33—37°C in geschmolzenem Hartfett suspendiert und homogenisiert und anschließend die Mischung in Hohlzellen ausgießt, wobei die Dosierungseinheit 0,1 bis 30 mg Wirkstoff enthält; oder daß man Verbindungen der Formel I oder deren Salze mit physiologisch unbedenklichen Säuren bei einer Temperatur zwischen 50 bis 120°C, gegebenenfalls in Gegenwart eines oder mehrerer Emulgatoren, mit mindestens einem der folgenden Stoffe homogenisiert: Paraffin, Vaseline, aliphatischer Alkohol mit 12 bis 25 C-Atomen, aliphatische Monocarbonsäure mit 15 bis 20 C-Atomen, Polyoxyethylenpolyolfettsäureester, und die erhaltene Mischung zwischen 50 und 120°C mit Wasser, gegebenenfalls unter Zusatz eines mehrwertigen niederen aliphatischen Alkohols und/oder Phenoxyethanol emulgiert; oder daß man Verbindungen der Formel I oder deren Salze mit physiologisch unbedenklichen Säuren in Wasser oder Pflanzenöl, gegebenenfalls in Gegenwart eines Emulgators, bei Temperaturen zwischen 30—100°C auflöst, und die so erhaltene Lösung mit soviel Wasser oder Pflanzenöl auffüllt, daß die Endlösung 0,01 bis 5 Gewichtsprozent an Wirkstoff der Formel I enthält.

Als Emulgatoren kommen zum Beispiel in Frage: nichtionogene Emulgatoren sowie ionogene Emulgatoren. Bei den nichtionogenen Emulgatoren handelt es sich beispielsweise um Triglyceridgemische von gesättigten Pflanzenfettsäuren mit $C_8$, $C_{10}$ und $C_{12}$ oder um Emulgatoren auf der Basis von Polyadditionsprodukten des Ethylenoxids, wie zum Beispiel alkyl- und acylsubstituierte Polyadditionsprodukte des Ethylenoxids, Polyethylenglykol-fettsäureester, Umsetzungsprodukte von Ethylenoxid mit Ricinusöl beziehungsweise hydriertem Ricinusöl, Ester von hydrierten Ricinusölfettsäuren mit oxyethyliertem Glycerin. Weiterhin kann es sich um Emulgatoren auf Basis von Fettsäureamiden oder Fettsäurekondensationsprodukten mit hydrophilen Gruppen handeln. Als ionogene Emulgatoren kommen zum Beispiel Emulgatoren auf Basis von Fettsäuremonoestern des Glycerins oder anderer mehrwertiger Alkohole (Lunacera alba) in Frage.

Beispiel 1

4-(p-(Clor-benzyl)-2-[-hexahydro-1-(p-methyl-benzyl)azepin-4-yl]-1-(2H)-phthalazinon

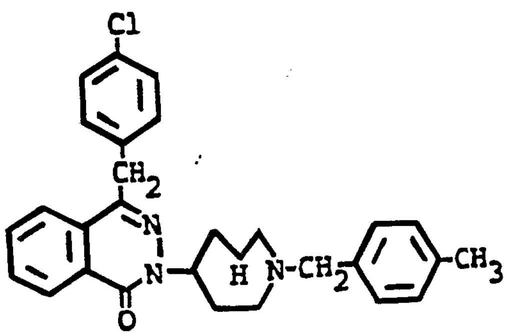

0,016 mol (7 g) 4-(p-Chlor-benzyl)-2-(hexahydroazepin-4-yl)-1-(2H)-phthalazinon-hydrobromid werden in 50 ml Dioxan bei 50°C gelöst. Zu dieser Lösung gibt man unter Rühren 0,048 mol (6,5 ml beziehungsweise 4,7 g) Triethylamin und 0,016 mol (2,1 ml beziehungsweise 2,2 g) 4-Methylbenzylchlorid. Danach wird 5 Stunden bei 85°C gerührt. Man läßt auf Raumtemperatur abkühlen, filtriert das Gemisch und engt die erhaltene Lösung am Rotationsverdampfer ein. Der dunkle, ölige Rückstand wird in propanolischer Salzsäure gelöst und durch Zusetzen von Ether ein zähöliges Produkt gefällt. Die überstehende Lösung dekantiert man ab. Der ölige Rest wird mit Ether überschichtet und über Nacht im verschlossenen Kolben bei Raumtemperatur stehen gelassen. Danach verreibt man die etwas gehärtete Substanz portionsweise mit Ether, saugt ab und trocknet an der Luft.

Abschließend löst man in warmen Methylethylketon, gibt Ether bis zur Trübung zu und läßt das Salz über Nacht auskristallisieren. Das abfiltrierte Produkt wird im Vakuum getrocknet. Ausbeute: 3,4 g (43 %) F. 191-195°C (Hydrochlorid).

Die Ausgangssubstanz wird zum Beispiel wie folgt erhalten:

60 g (0,157 mol) 4-(p-Chlorbenzyl)-2-(hexahydro-1-methyl-azepin-4-yl)-1-(2H)-phthalazinon werden unter Erwärmung auf 95°C in 600 ml trockenem Toluol gelöst. Anschließend werden 51,1 g (0,471 mol = 45 ml) Chlorameisensäureethylester in 45 ml Toluol unter Rühren zugetropft. Die Mischung wird 5 Stunden bei 95°C gerührt. Nach dem Abkühlen auf Raumtemperatur wird das Reaktionsgemisch von Unlöslichem

abgesaugt und am Rotationsverdampfer eingeengt. Es verbleibt ein öliger Rückstand, der mit wenig Ether verrieben als weißes kristallines Produkt anfällt und bei 103-105°C schmilzt (Ausbeute: 53,4 g (77 %).

53,4 g (0,12 mol) des so erhaltenen 1-Carbethoxy-Derivats (Formel I, $R_5$ im Rest A = $COOC_2H_5$) und 114 ml einer 40 %igen Lösung von Bromwasserstoff in Eisessig werden unter intensivem Rühren 4 Stunden auf 85—90°C erhitzt; bei zunehmender Erwärmung geht die Carbethoxyverbindung in Lösung. Nach dem Abkühlen wird die Lösung im Vakuum eingeengt. Aus dem erhaltenen, zähen, öligen Rückstand erhält man durch Umkristallisation aus Methanol die Ausgangsverbindung der Formel I, worin $R_5$ Wasserstoff ist, in Form des weißen kristallinen Hydrobromids.

Man saugt ab, wäscht mehrfach mit Methanol und trocknet im Vakuum. Ausbeute: 51 g (95 %) F.: 138—140°C.

Zur Darstellung der freien Base wird das Hydrobromid in 300 ml destilliertem Wasser aufgeschlämmt und mit 100 ml konzentriertem Ammoniak alkalisch gestellt. Nach 2stündigem Rühren wird abgesaugt und mit Wasser gewaschen, bis die Waschlösung neutral bleibt. Das weiße Produkt wird im Vakuumexsikator über $P_2O_5$ mehrere Tage getrocknet. Ausbeute: 125 g (77 % der Theorie) F. 123—125°C.

Analog Beispiel 1 werden die Verbindungen der Formel I erhalten, die in der Tabelle 1 aufgeführt sind. Falls nichts anderes angegeben ist, sind in Spalte 4 die Schmelzpunkte der entsprechenden Hydrochloride angegeben. Bei den Beispielen 2 bis 8 sind die Molmengen an den Ausgangsverbindungen II und III jeweils dieselben; bei den Beispielen 9, 10, 11 und 13 werden jeweils 0,027 mol und bei Beispiel 12 0,015 mol Ausgangsverbindung II eingesetzt. Bei den Beispielen 5 bis 11 sowie 13 wird die Ausgangsverbindung in Form der freien Base eingesetzt, bei den Beispielen 1 bis 4 und 12 als Hydrobromid.

Die Aufarbeitung des Reaktionsproduktes nach Einengen beziehungsweise Entfernung der Reaktionslösung erfolgt bei den Beispielen 6, 7, 10 und 13 analog Beispiel 1.

Bei den Beispielen 2 und 8 wird der nach Eindampfen der Reaktionsmischung erhaltene Rückstand (nachdem er durchkristallisiert ist) vor der Salzbildung mit isopropanolischer HCl aus Isopropanol, bei Beispiel 3 aus Methanol und bei Beispiel 5 aus Ethanol umkristallisiert.

Bei den Beispielen 4, 9, 11 und 12 wird der Eindampfrückstand der Reaktionsmischung(gegebenenfalls nach Verrühren mit Isopropanol und Ether) zunächst über eine Kieselgelsäure gereinigt und dann nach Entfernung des Elutionsmittel mit isopropanolischer HCl das Hydrochlorid hergestellt. Elutionsmittel ist: Dichiormethan/Methanol/Ammoniak 25%ig = 85/15/1.

Im Falle von Beispiel 12 wird auch nach der chromatographischen Reinigung die Base in Aceton gelöst und durch Zugabe von in Aceton gelöster wasserfreier Oxalsäure des Oxalat erhalten, im Falle von Beispiel 9 wird der Rückstand nach der Chromatographie nicht in ein Salz überführt.

Um die Salze kristallin zu erhalten ist es gegebenenfalls ganz allgemein zweckmäßig, wiederholt mit Ether oder auch Methylethylketon zu verreiben; manchmal empfiehlt sich auch die Freisetzung der Base mit konzentriertem Ammoniak, Ausschütteln mit Ether, Trocknen und nochmalige Salzbildung mit isopropanolischer HCl in Isopropanol oder Methylethylketon und Ausfällung des Salzes durch Etherzusatz (Beispiel 13). Die erhaltenen Salze können dann auch noch aus einem geeigneten Lösungsmittel (z.B. Isopropanol) umkristallisiert werden.

Die Ausgangsverbindung II für die Beispiele 2 bis 4 sowie 9 bis 13 ist jeweils 4-(p-Chlor-benzyl)-2(hexahydro-azepin-4-yl)-1-(2H)-phthalazinon. Die Ausgangsverbindung II (4-(p-Fluor-benzyl)-2(hexahydro-azepin-4-yl)-1-(2H)-phthalazinon) für die Beispiele 5—8 kann beispielsweise wie folgt erhalten werden: 50 g (0,136 mol) gut getrocknetes 4-(Fluorbenzyl)-2-(hexahydro-1-methyl-azepin-4-yl)-1-(2H)-phthalazinon werden in 400 ml Toluol durch Erwärmen auf 80° C gelöst. Sollten sich Wassertropfen an der Kolbeninnenwand abscheiden, wird dieses Restwasser mit Hilfe eines Wasserabscheiders azeotrop abdestilliert.

Anschließend werden 44,5 g (0,408 mol = 38 ml) Chlorameisensäureethylester zugetropft und 1½ Stunden nachgerührt. Nach dem Abkühlen auf Raumtemperatur wird von unlöslichen Bestandteilen abgesaugt und die erhaltene Lösung im Vakuum eingeengt. Den zurückbleibenden Sirup, der von selbst durchkristallisiert, verreibt man mit Ether. Das weiße kristalline Produkt wird abgesaugt, mit Ether gewaschen und getrocknet. Ausbeute: 33 g (57%) F. 95—96°C.

Eine Mischung aus 33 g (0,078 mol) der so erhaltenen Verbindung und 65 ml 40%igen HBr/Eisessig wird langsam auf 100°C Ölbadtemperatur erhitzt und 2 Stunden gerührt. Man läßt abkühlen und engt die Lösung am Rotationsverdampfer ein. Der ölige Rückstand wird in 250 ml destilliertem $H_2O$ gelöst und die Lösung durch Zusetzen von 40 ml konzentriertem Ammoniak alkalisch gestellt. Nachfolgend fällt die Base als kristalliner Niederschlag aus, wird abgesaugt und im Exsikkator über Phosphorpentoxid getrocknet. Ausbeute: 23g (48%) F. 86—88°C.

Das 4-(Fluorbenzyl)-2-(hexahydro-1-methyl-azepin-4-yll-(2H)-phthalazinon wird zum Beispiel wie folgt erhalten: 230 g (0,809 Mol) N-Benzoyl-N'-(1-methyl-hexahydroazepin-4-yl)-hydrazin werden 4 Stunden mit 830 ml 23 %iger Salzsäure unter Rückfluß erhitzt (Benzoesäure fällt aus). Man kühlt ab und engt am Rotationsverdampfer ein, gibt ca. 200 ml Toluol zu und engt erneut ein. Der Rückstand wird mit 209 g 2-(4-Fluor-phenacetyl)-benzoesäure, 149,5 g KOH 85 %ig und 2300 ml Methanol 2½ Stunden gekocht. Man engt das Gemisch ein und verrührt den Rückstand mit 3,3 Liter $H_2O$ und 785 ml 2molarer NaOH. Die Base fällt aus. Man rührt nach, saugt ab und wäscht mit $H_2O$ neutral. Bei 50°C im Vakuum wird getrocknet.

Tabelle 1

| Beispiel Nr. | $R_1$ $R_2$=H $R_3$=H | A= 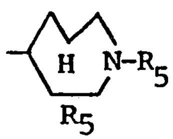 | F. (Hydrochlorid) °C | Ausbeute in % | Ausgangsstoff III $Cl-R_3$ in mol | Triethylamin in mol | Reaktionstemperatur/ Dauer (in Stunden) |
|---|---|---|---|---|---|---|---|
| 2 | p - Cl | 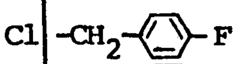 | 145 | 68 | 0,016 | 0,048 | 90° C / 5 |
| 3 | p - Cl | 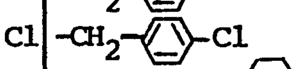 | 204 - 7 | 30 | 0,013 | 0,032 | 95° C / 4 |
| 4 | p - Cl | 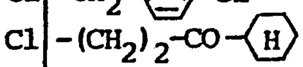 | 140 | 25 | 0,0156 | 0,47 | 85° C / 5 |
| 5 | p - F | 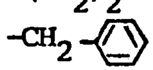 | 197 - 199 | 65 | 0,02 | 0,04 | 85 - 90° C / 3 |
| 6 | p - F | $-CH_2-CH=CH_2$ | 180 - 182 | 64 | 0,02 | 0,04 | 75° C / 3 |
| 7 | p - F | 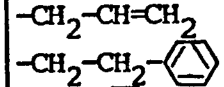 | 212 - 215 | 40 | 0,014 | 0,028 | 95° C / 4 |
| 8 | p - F | 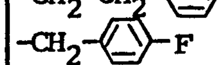 | 197 - 200 | 45 | 0,014 | 0,028 | 80° C / 3 |
| 9 | p - Cl | $-CO-CH=CH-C_6H_5$ | 204 - 206 Base | 55 | 0,054 | 0,081 | 20 - 23° C / 8 |
| 10 | p - Cl | $-CH_2-CH=CH-C_6H_5$ | 224 - 226 Zersetzung | 69 | 0,054 | 0,081 | 101° C / 13 |
| 11 | p - Cl | 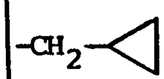 | 226 - 228 Zersetzung | 53 | 0,054 | 0,081 | 101° C / 13 |
| 12 | p - Cl | $-(CH_2)_2-O-(CH_2)_2-OH$ | 112 - 116 | 49 | 0,018 | 0,027 | 101° C / 8 |
| 13 | p-Cl | $-(CH_2)_2-N(CH_3)_2$ | Oxalat 248-250 (2HCl) | 44 | 0,054 | 0,054 | 101°C/4 |

Beispiele 14 bis 43 (Tabelle 2 und 3)

Die Herstellung von Verbindungen I gemäß den Beispielen 14—38 erfolgt gemäß der folgenden Arbeitsvorschrift:

Zu 1 mol Ausgangsverbindung II (freie Base) gibt man bei Zimmertemperatur 10 mol Dioxan, 3 mol Triethylamin und 2 mol der Ausgangsverbindung III, erhitzt das Reaktionsgemisch mehrere Stunden bei 100°C und gießt dieses nach dem Einengen in destilliertes Wasser. Die weitere Aufarbeitung erfolgt nach der Methode A oder B.

Methode A: Das in Wasser unlösliche Öl wird 2 mal mit Ether, die vereinigten etherischen Extrakte 1 mal mit Wasser ausgeschüttelt und über $Na_2SO_4$ getrocknet. Nach dem Abdampfen wird das zurückbleibende Öl in einem Isobutylmethylketon (oder auch Ethylmethylketon)-Toluol-Gemisch aufgenommen, mit isopropanolischer Salzsäure angesäuert und gegebenenfalls etwas eingeengt. Durch Animpfen der heißen Lösung mit im Reagenzglas erzeugten Kristallen (und/oder Etherzusatz) wird ein kristallines Produkt erhalten.

Methode B: Das in Wasser ungelöste Öl wird 3 mal mit Methylenchlorid, die vereinigten organischen Phasen 2 mal mit Wasser ausgeschüttelt. Die über $Na_2SO_4$ getrocknete Lösung wird am Reaktionsverdampfer eingeengt, der Rückstand mit 50—70 ml Aceton aufgenommen und mit 20 g Kieselgel verrührt (dient zur Adsorption von Ausgangsmaterial). Nach 10minütigem Rühren wird vom Kieselgel abgesaugt, um das Filtrat einzuengen. Den Abdampfrückstand nimmt man in Methylketon/Toluol auf, säuert mit isopropanolischer HCl an und engt die Lösung bis zur bleibenden Trübung ein. Gegebenenfalls wird durch Etherzusatz die Kristallisation begünstigt. Das anfallende Rohprodukt wird abgesaugt, mit Aceton gewaschen und mit $CH_2Cl_2$/Methylketon ausgekocht. Nach mehrstündigem Stehen saugt man das reine Produkt ab, wäscht mit viel Aceton und Diethylether, trocknet die Kristalle im Vakuum bei 50°C.

Für die Beispiele 14 bis 24 wird eine Ausgangsverbindung der Formel II eingesetzt, worin $R_1$ ein Chloratom in p-Stellung ist, für die Beispiele 25 bis 43 eine Ausgangsverbindung II, worin $R_1$ ein Fluoratom in p-Stellung ist. In Tabelle 2 ist A stets der Rest

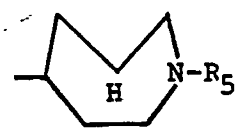

in Tabelle 3 stets der Rest

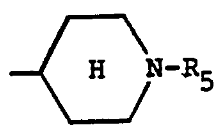

Die Ausgangsstoffe der Formel III für die Beispiele 14 bis 43 sind Chloride der Formel $C_1$—$R_5$, wobei $R_5$ die in Tabelle 2 beziehungsweise Tabelle 3 in Spalte 2 angegebenen Bedeutungen hat.

Der Buchstabe Z hinter der Schmelzpunkttemperatur bedeutet, daß die betreffende Substanz unter Zersetzung schmilzt.

Die Verfahrensprodukte gemäß den Beispielen 16, 19, 22, 24, 31 und 33 von Tabelle 2 sowie Beispiel 37 von Tabelle 3 enthalten je ein Molekül Kristallwasser.

Die Herstellung von Verbindungen I gemäß den Beispielen 39—43 erfolgt (siehe Tabelle 3) nach folgender Arbeitsvorschrift (Die Reaktionstemperatur und die Lösungsmittelmenge ist hier in der letzten Spalte von Tabelle 3 angegeben):

0,05 Mol der Ausgangssubstanz II (freie Base) werden in das Lösungsmittel eingetragen und bei 40—50°C unter Rühren gelöst. Es erfolgt die Zugabe des Protonenfängers ($K_2CO_3$ oder Triethylamin) und das Zutropfen der Ausgangsverbindung III ($C_1$—$R_5$). Das Reaktionsgemisch wird mehrere Stunden unter Rückfluß gekocht oder bei einer bestimmten Temperatur gerührt. Nach dem Abkühlen auf Zimmertemperatur werden die ausgefallenen Salze abgesaugt und das Filtrat am Rotationsverdampfer eingeengt. Der Rückstand wird aufgearbeitet und das entstandene Produkt durch Umkristallisation oder Säulenchromatographie gereinigt. In den meisten Fällen schließt sich eine Salzbildung zur Löslichkeitsverbesserung an. Zur Gewinnung des Hydrochlorids wird die Base in Ethylmethylketon oder Isopropanol aufgeschlämmt, mit isopropanolischer HCl bis zur sauren Reaktion versetzt und mit Diethylether bis zur ersten leichten Trübung verrührt. Das entsprechende Oxalat erhält man durch Lösen der Substanz in Aceton und Zugabe von ebenfalls in Aceton gelöster wasserfreier Oxalsäure. Bei den Beispielen 39, 40, 41 und 43 wird als Lösungsmittel Dioxan und als basische Verbindung Triethylamin verwendet (bei Beispiel 43 0,15 Mol Triethylamin, sonst 0,1 Mol).

Bei Beispiel 42 wird als Lösungsmittel Dimethylacetamid und als basische Verbindung 0,1 Mol $K_2CO_3$ verwendet. Die Menge an Ausgangsverbindung III $R_5$—$C_1$ bei den Beispielen 39-41 ist 0,06 Mol, bei den Beispielen 42 und 43 jeweils 0,1 Mol.

Aufarbeitung: Bei den Beispielen 39 bis 41 wird der Rückstand nach Eindampfen der Reaktionsmischung aus Ethanol (50—250 $m^3$) unter Kohlezusatz (bei Beispiel 41 Kieselgur-Zusatz) umkristallisiert und anschließend das Hydrochlorid hergestellt. Bei den Beispielen 42 und 43 wird der ölige Rückstand mit jeweils 50 ml Diethylether verrührt wobei Kristallisation erfolgt; bei Beispiel 43 schließt sich eine Chromatographie über eine Kieselgelsäule an (Elutionsmittel: Dichlormethan/Methanol/Ammoniak 25%ig = 85/15/1).

Bei den Beispielen 14—43 ist $R_2$ und $R_3$ stets Wasserstoff.

Tabelle 2

| Beispiel Nr. | 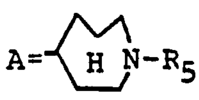 $R_5$ | F. °C Hydrochlorid | Ausbeute in % | Reaktionszeit (in Stunden) | Methode der Aufarbeitung |
|---|---|---|---|---|---|
| 14 | 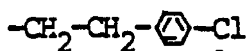 | 218–223 | 20 | 10 | B |
| 15 | 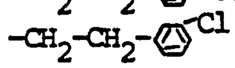 | 210–214 | 34 | 8 | A |
| 16 | 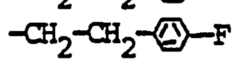 | 180–186 | 37 | 12 | B |
| 17 | 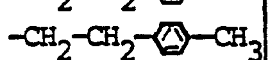 | 225–231 | 49 | 12 | B |
| 18 | 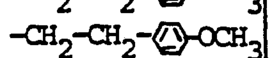 | 205–208 | 36 | 16 | B |
| 19 | 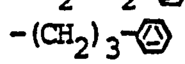 | 192–195 | 77 | 5 | B |
| 20 | 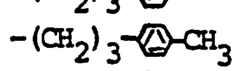 | 207–212 Z | 80 | 1 | A |
| 21 | 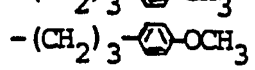 | 228–234 Z | 33 | 5 | A |
| 22 | 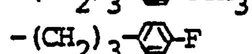 | 204–207 | 90 | 2 | B |
| 23 | 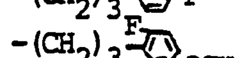 | 189–194 | 33 | 6 | A |
| 24 | 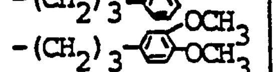 | 123–135 Z | 20 | 6 | B |
| 25 | 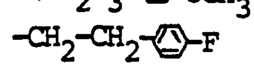 | 194–197 | 30 | 10 | B |
| 26 | 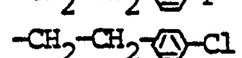 | 215–219 Z | 33 | 10 | A |
| 27 | 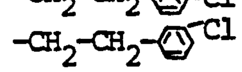 | 215–218 Z | 34 | 3 | A |
| 28 | 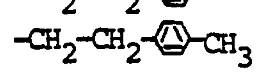 | 241–244 Z | 30 | 8 | A |
| 29 | 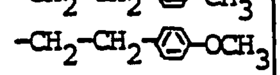 | 182–186 Z | 23 | 10 | A |
| 30 | 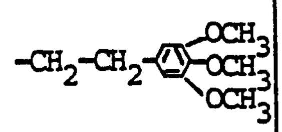 | 181–186 Z | 46 | 16 | B |
| 31 | 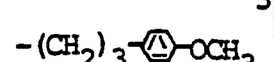 | 185–191 Z | 50 | 4 | B |
| 32 | 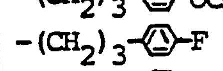 | 207–211 | 29 | 4 | B |
| 33 | 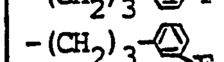 | 190–193 | 15 | 4 | B |
| 34 | 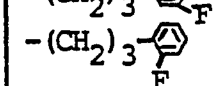 | 189–191 | 69 | 6 | B |

Tabelle 3

| Beispiel Nr. | A= 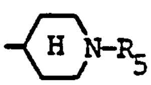 $R_5$ | F. °C Hydro-chlorid | Ausbeute in % | Reaktions-zeit in Stunden | Methode der Aufarbei-tung |
|---|---|---|---|---|---|
| 35 | $-(CH_2)_3-$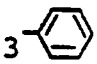 | 209–211 | 90 | 5 | A |
| 36 | $-(CH_2)_3-$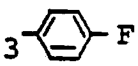 | 191–195 | 62 | 2 | B |
| 37 | $-(CH_2)_3-$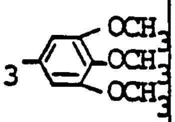 | 172–176 | 64 | 13 | B |
| 38 | $-(CH_2)_2-$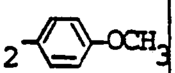 | 191–195Z | 63 | | B |
| | | | | | Menge Lösungs-mittel/ Reaktions-temperatur (siehe Seite 37) |
| 39 | $-CH_2-$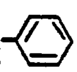 | 219–221 | 53 | 3 | 120/85–90°C |
| 40 | $-CH_2-CH=CH_2$ | 157–158 | 57 | 3 | 120/70°C |
| 41 | $-(CH_2)_2-$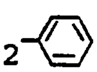 | 246–248 | 59 | 8 | 120/101°C |
| 42 | $-(CH_2)_2-OCH_3$ | 180–183 Oxalat | 46 | 6 | 115/120°C |
| 43 | 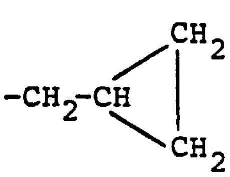 | 195–197 | 15 | 16 | 70/101°C |

Beispiele 44-49

Die Herstellung von Verbindungen der Formel I ($R_1$= Fluor in 4-Stellung; $R_2$ und $R_3$ = H; A siehe Tabelle 4 gemäß den Beispielen 44—49 erfolgt nach folgender Arbeitsvorschrift:

0,011 Mol der Ausgangssubstanz II (freie Base) werden in 50 ml Dioxan eingetragen (30 ml Dioxan bei Beispiel 48; 25 ml Dioxan bei Beispiel 49) und bei 40—50°C unter Rühren gelöst. Es erfolgt Zugabe von

jeweils 0,033 Mol Triethylamin (bei Beispiel 49 0,013 Mol) und das Zutropfen der Ausgangsverbindung III ($C_1$—$R_2$). Das Reaktionsgemisch wird mehrere Stunden unter Rückfluß gekocht oder bei einer bestimmten Temperatur gerührt. Nach dem Abkühlen auf Zimmertemperatur werden die ausgefallenen Salze abgesaugt und das Filtrat am Rotationsverdampfer eingeengt. Der Rückstand wird aufgearbeitet und das entstandene Produkt durch Umkristallisation (Beispiele 44, 45, 47 und 48) oder Säulenchromatographie (Beispiel 46) gereinigt. Elutionsmittel: Dichlormethan/Methanol/Ammoniak 25% = 85/15/1. In den meisten Fällen schließt sich eine Salzbildung zur Löslichkeitsverbesserung an. Zur Gewinnung des Hydrochlorids wird die Base in Ethylmethylketon oder Isopropanol aufgeschlämmt, mit isopropanolischer HCl bis zur sauren Reaktion versetzt und mit Diethylether bis zur ersten leichten Trübung verrührt.

Tabelle 4

| Beispiel Nr. | A = 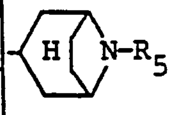 $R_5$ | F. (Hydrochlorid) °C | Ausbeute in % | Ausgangsstoff III $Cl-R_2$ in Mol | Reaktionstemperatur/ Dauer (in Stunden) |
|---|---|---|---|---|---|
| 44 | $CH_2-CH=CH_2$ | 135–140 sintert bei 130°C | 73 | 0,033 | 101°C/8 |
| 45 | $CH_2$- 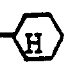 | 156–157 (Base) | 45 | 0,047 | 101°C/24 |
| 46 | 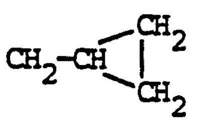 | 152–160 | 26 | 0,054 | 101°C/32 |
| 47 | $-(CH_2)-CO-$ 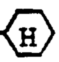 | 143–146 | 56 | 0,033 | 101°C/6 |
| 48 | $-(CH_2)_2-$ 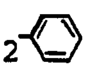 | 245–247 | 30 | 0,033 | 101°C/10 |
| 49 | $-CH_2-$ 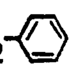 $R_1$=p-Cl | 230–232 | 53 | 0,0065 | 90°C/6 |

Beispiele für das Verfahren b):

Beispiel 50

4-(4-Fluor-benzyl)-2-(hexahydro-1-benzyl-azepin-4-yl)-1-(2H)-phthalazinon

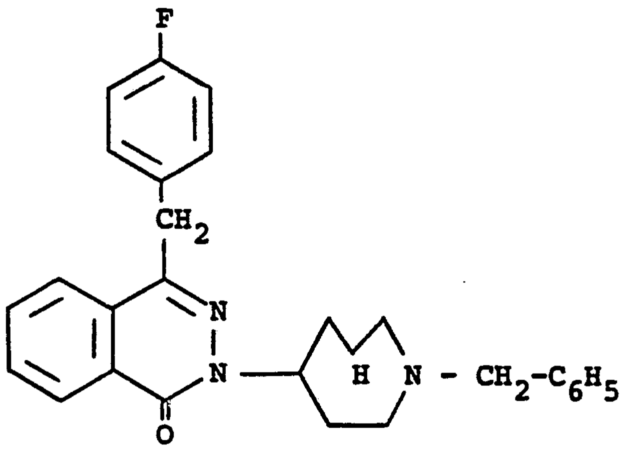

34 g (0,088mol) 1-Benzoyl-2-(hexahydro-1-benzylazepin-4-yl)-hydrazin × HCl werden zusammen mit 90 ml 23 %iger HCl 4 Stunden unter Rückfluß und Rühren erhitzt. Nach dem Abkühlen rotiert man das Reaktionsgemisch im Vakuum zur Trockene ein. Dem verbleibenden Rückstand werden 22,7 g (0,088 mol) 2-(p-Fluor-phenacetyl)-benzoesäure und 14 g (0,25 mol) Kaliumhydroxid, gelöst in 250 ml Methanol, zugesetzt. und die Lösung 2 Stunden bei Raumtemperatur nachgerührt. Anschließend destilliert man das Solvens im Vakuum ab und versetzt den Rückstand mit 500 ml $H_2O$ und 2N NaOH. Unter Rühren fällt das Reaktionsprodukt in Form der Base als kristalliner Niederschlag aus, der abfiltriert und gründlich mit $H_2O$ nachgewaschen wird. Das Produkt wird im Exsikkator über Phosphorpentoxid getrocknet und aus Isopropanol umkristallisiert. Ausbeute: 26 g, F. 197—199°C.

Die Ausgangssubstanzen werden zum Beispiel wie folgt erhalten.

1-Benzoyl-2-(hexahydro-1-benzyl-azepin-4-yl)-hydrazin × HCl

Die Mischung aus 68,3 g (0,27 mol) N-Benzylazepin-on-(4)-hydrochlorid, 38 g (0,28 mol) Benzoylhydrazin und 250 ml Methanol wird 1,5 Stunden bei 30°C bis zur Lösung gerührt. Nach dem Abkühlen auf 4°C wird unter Einhaltung dieser Temperatur (Eiskühlung) 15,3 g (0,27 mol) Kaliumhydroxid, gelöst in 150 ml Methanol, unter Rühren zugetropft. Nach erfolgter Zugabe werden bei einer Innentemperatur von 14—18°C 15,8 g (0,42 mol) Natriumborhydrid im Verlauf von 1 Stunde in kleinen Portionen eingetragen (exotherme Reaktion; starkes Aufschäumen wegen $H_2$-Entwicklung) und das Reaktionsgemisch bei Raumtemperatur 2 Stunden nachgerührt. Anschließend stellt man durch Zugabe von 120 ml isopropanolischer Salzsäure das Reaktionsgemisch auf pH 5 ein und rührt 2 Stunden bei Eis/Wasser Kühlung Nach dem Ab filtrieren der anorganischen Salze wird das Filtrat im Vakuum eingeengt. Das zurückbleibende Öl wird mit 250 ml Aceton/Methanol = 10:1 versetzt. Unter Rühren fällt das Hydrochlorid als kristallines Produkt aus. Der Niederschlag wird abgesaugt und getrocknet. Ausbeute: 27 g (35 %), F. 176—180°C.

2-(p-Fluor-phenacetyl)-benzoesaure

In 410 ml (3,9 mol) 29 %ige Natronlauge trägt man 250 g (1,04 mol) p-Fluor-benzylidenphthalid bei einer Ölbadtemperatur von 70°C unter Rühren ein. Gegen Ende der Zugabe wird die Ölbadtemperatur auf 110°C erhöht. Es setzt sich dabei ein rotgefärbtes Öl ab. Beim Abkühlen fällt das Öl als kristallines Produkt an. Diese Substanz wird in 500 ml destilliertem $H_2O$ gelöst (rot gefärbte Lösung) und diese Lösung unter Rühren durch Zutropfen von 340 ml konzentrierter HCl sauer eingestellt. Der ausgefallene Niederschlag wird abgesaugt, mehrmals mit destilliertem $H_2O$ gewaschen und im Vakuum getrocknet. Anschließend wird aus Toluol umkristallisiert Ausbeute. 220 8 g (82 %) F. 146 150°C.

Analog dem vorangegangenen Beispiel können aus jeweils 0,088 Mol der durch die Reste $R_1$, $R_2$, $R_3$ und $R_4$ substituierten Phenacetylbenzoesäure (zum Beispiel 2-(p-Chlor-phenacetyl)-benzoesäure beziehungsweise 2-(p-Fluor-phenacetyl)-benzoesäure) und 0,088 Mol eines Hydrazins der Formel

$$H_2N—NH—A$$

ebenfalls die Verbindungen der Formel I erhalten werden (zum Beispiel die Verbindungen, die in den Tabellen 1—4 aufgeführt sind). Das Hydrazin wird hierbei zum Beispiel in Form des Acylderivats (Benzoyl- oder Acetylderivat) eingesetzt, aus welchem vor der eigentlichen Umsetzung durch Abspaltung der Acylgruppe das Hydrazin freigesetzt wird.

Die Hydrochloride können zum Beispiel durch Behandeln der entsprechenden Base mit Isopropanol/HCl, anschließende Fällung mit Ether und Umkristallisation aus Methylethylketon + Ether erhalten werden.

Beispiel 51

4-(3,4-Dichlorbenzyl)-2-(hexahydro-1-benzyl-azepin-4-yl)1-(2H)-phthalazinon

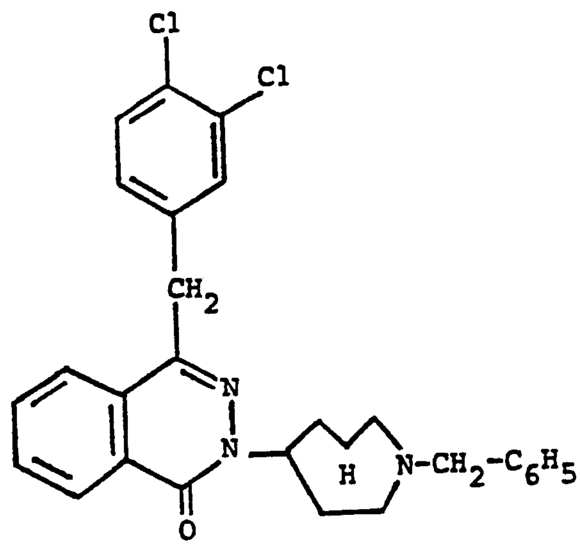

Eine Lösung von 3,28 g (8,15 mmol) 4-(3,4-Dichlorbenzyl)-2-(hexahydro-1H-azepin-4-yl)-1-(2H)-phthalazinon, 3,4 ml (29,5 mmol) Benzylchlorid und 9 ml (64,6 mmol) Trietylamin in 20 ml Dioxan wird 12 Stunden bei 200°C erhitzt.

Danach wird eingeengt, der Rückstand mit Wasser versetzt und Dichlormethan ausgeschüttelt. Die organische Phase wird mit $MgSO_4$ getrocknet, filtriert und eingeengt. Das zurückbleibende Öl kristllisiert über Nacht. Die Kristalle werden in Ether aufgeschlämmt und verrührt. Nach Absaugen und Waschen der Substanz mit Ether wird die Lösung einrotiert.

Es resultiert eine farblose kristalline Substanz vom F. 117 118°C. Ausbeute 0,55 g (14%).

Beispiel 52

4-(3,4-Dichlorbenzyl)-2-(hexahydro-1-phenethyl-azepin4-yl)-1-(2H)-phthalazinon

3,36 g (8,35 mmol)4-(3,4 Dichlorbenzyl)-2-(hexahydro-1H-azepin-4-yl)-1-(2H)-phthalazinon 4 ml (29 3 mmol), Phenethylbromid und 4,5 ml (32,20 mmol) Triethylamin werden in 20 ml Dioxan gelöst.

Das Reaktionsgemisch wird für 12 Stunden bei 100°C unter Rühren erhitzt.

Es wird einrotiert, der Rückstand mit Wasser versetzt, Dichlormethan eluiert, mit $MgSO_4$ getrocknet, filtriert, eingeengt und das resultierende Öl über eine Kieselgelsäule chromatografiert. Die aus den entsprechenden Eluaten gewonnene Base wird in das Oxalat überführt.

Ausbeute 0,39 g (ca. 8%) F. 161—162°C.

Beispiel 53

4-(4-Methylbenzyl)-2-(hexahydro-1-benzyl-azepin-4-yl)-1-(2H)-phthalazinon

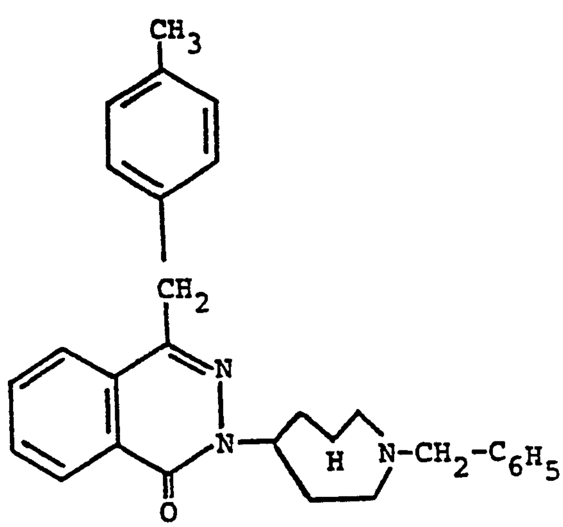

3,02 g (8,69 mmol) 4-(4-Methylbenzyl)-2-(hexahydro-1H-azepin-4-yl)-1-(2H)-phthalazinon, 3 ml (26,00 mmol) Benzylchlorid und 3,6 ml (25,8 mmol) Triethylamin werden in 20 ml Dioxan gelöst.

Das Reaktionsgemisch wird für 10 Stunden unter Rühren bei 100°C erhitzt. Danach wird eingeengt, der Rückstand mit Wasser versetzt und mit Dichlormethan ausgeschüttelt. Die Lösung wird mit $MgSO_4$ getrocknet, filtriert und eingeengt. Der ölige Rückstand wird über eine Kieselgelsäule chromatografiert.

Nach Einengen des Eluates resultiert ein kristallines Produkt, welches in Ether aufgeschlämmt, verrieben und abgesaugt wird.

Ausbeute: 1 g (26,3%) F. 128—129°C.

Beispiel 54

4-(Fluor-benzyl)-2-1-[2-(3-trifluormethyl-phenyl)ethyl]-hexahydroazepin-4-yl-1-(2H)-phthalazinon

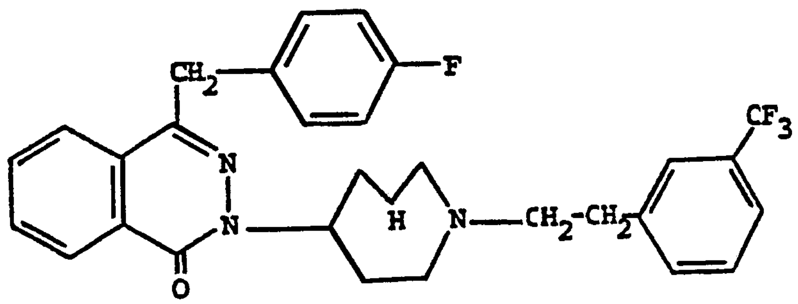

Zu 80 ml Dioxan gibt man 10,5 g (0,03 Mol) 4-(4-Fluorbenzyl-2-(hexahydro-azepin-4-yl)-1-(2H)-phthalazinon, 9,1 g Triethylamin und 5 Tropfen Dimethylformamid, tropft zu dieser Mischung bei Raumtemperatur 10 g (0,04 Mol)2-[3-Trifluormethyl-phenyl]-ethylhromid zu und erhitzt 6 Stunden unter Rückfluß Nach dem Abkühlen saugt man ab. Die Lösung wird im Rotationverdampfer eingeengt und in 150 ml $CHCl_3$ gelöst und 2 mal mit je 50 ml $H_2O$ extrahiert. Die $CHCl_3$-Phase wird über $MgSO_4$ getrocknet, abfiltriert und eingeengt. Der Rückstand wird mit Ether verrieben über Nacht stehenlassen abgesaugt mit Ether nachgewaschen und, im Vakuum bei 40°C getrocknet. In Isopropanol wird mit ätherischer Salzsäure das Hydrochlorid hergestellt und aus der 40 fachen Menge Aceton umkristallisiert. Das Hydrochlorid schmilzt bei 184—187°C (Ausbeute 6,1 g).

Beispiele für pharmazeutische Zubereitungen

Tabletten, 5 m Wirkstoff

50 g Wirksubstanz nach Beispiel 5, 450 g Lactose, 150 g Maisstärke und 10 g hochreines amorphes Siliciumdioxid werden durch ein Sieb (Maschenweite 0,8 mm) gegeben, gemischt und mit 200 g einer 5 %igen wässrigen Gelatinelösung in der Wirbelschicht granuliert. Das trockene Granulat, 242 g mikrokristalline Cellulose, 80 g Maisstärke und 8 g Magnesiumstearat werden gesiebt (0,8 mm Maschenweite), homogen gemischt und in üblicher Weise zu Tabletten von 100 mg Gewicht und einem Durchmesser von 6 mm gepreßt. 1 Tablette enthält 5 mg Wirkstoff.

Kapseln, 5 m Wirkstoff

50 g Wirkstoff nach BeisPiel 7, 50 g Maisstärke, 1070 g Calciumhydrogenphosphat-dihydrat und 10 g hochreines amorphes Siliciumdioxid werden durch ein Sieb (0,8 mm Maschenweite) gegeben, homogenisiert und in der Wirbelschicht mit 200 g einer 10 %igen wässrigen Gelatinelösung granuliert. 200 g Maisstärke und das trockene Granulat werden gesiebt (0,8 mm Maschenweite), homogenisiertund in üblicher Weise zu 140 mg in Hartgelatinekapseln der Größe 3 dosiert. 1 Kapsel enthält 5 mg Wirkstoff

Wasser-in-Öl-Creme, 1 % Wirkstoff

10 g mikronisierte Wirksubstanz entsprechend Beispiel 5 werden in einer Schmelze (70°C) aus 250 g weißer Vaseline, 20 g eines Emulgators auf Basis von Fettsäuremonoestern des Glycerins oder anderer mehrwertiger Alkohole (Lunacera alba), 5 g Cetylalkohol und 30 g eines nichtionogenen Emulgators (Cremophor® Wasserin-Öl-7) mit Hilfe eines hochtourigen Dispergiergerätes verteilt. In diese Schmelze wird eine 70°C warme Mischung aus 10 g Phenoxyethanol, 20 g Glycerin und 655 g Wasser unter Verwendung eines hochtourigen Emulgiergerätes eingearbeitet und die Emulsion bis 30°C unter Rühren abgekühlt. 1 g Creme enthält 10 mg Wirkstoff.

Öl-in-Wasser-Creme, 1 % Wirkstoff

10 g mikronisierter Wirkstoff nach Beispiel 7 wird in 100 g dünnflüssigem Paraffin suspendiert und in eine Schmelze (70°C) aus 60 g eines Polyoxyethylenpolyolfettsäureesters, 20 g einer Mischung von Cetylalkohol und Stearylalkohol (1:1) 40 g Stearinsäure und 55 g eines Lösungsvermittlers auf Basis eines Triglyceridgemisches von gesättigten pflanzenfettsäuren $C_8$, $C_{10}$ und $C_{12}$ (Miglyol® 812) eingearbeitet. Die wäßrige Phase besteht aus 10 g Phenoxyethanol, 30 g Glycerin, 655 g Wasser und 20 g 1N-Natronlauge. Bei

60°C werden beide phasen mit Hilfe eines hochtourigen Dispergierge rätes emulgiert und kaltgerührt 1 g Creme enthält 10 mg Wirkstoff.

**Patentansprüche für die Vertragsstaaten: BE CH DE FR GB IT LI LU NL SE**

1. 4-Benzyl-1-(2H)-phthalazinon-Derivate der Formel

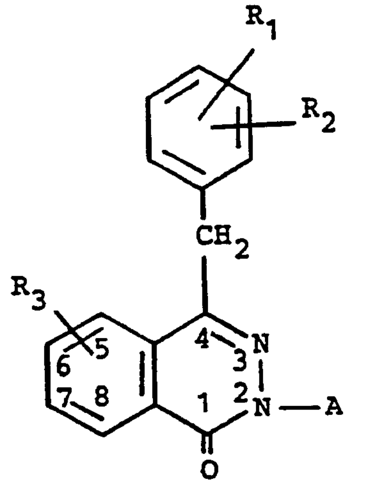

I

worin $R_1$ Fluor, Chlor, Brom, Trifluormethyl, $C_1$—$C_6$-Alkyl, Hydroxy, $C_1$—$C_6$-Alkoxy, $C_2$—$C_6$-Alkanoyloxy, Amino, $C_1$—$C_6$-Alkylamino, Di-$C_1$—$C_6$-alkylamino, $C_2$—$C_6$-Alkanoylamino, $C_2$—$C_6$-Alkanoyl-$C_1$—$C_6$-alkylamino oder eine Nitrogruppe bedeutet, $R_2$ Wasserstoff ist oder eine der für $R_1$ angegebenen Bedeutungen hat, wobei $R_1$ und $R_2$ gleich oder verschieden sein können, der Rest $R_3$ Wasserstoff, $C_1$—$C_6$-Alkyl oder Hydroxy bedeutet und

A den Rest 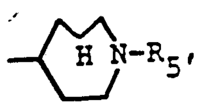

den Rest 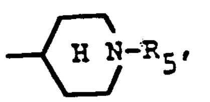

den Rest 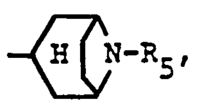

den Rest 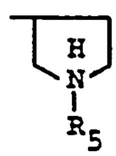 oder

den Rest 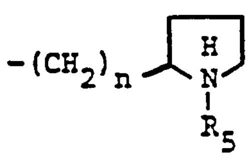

mit n = 1 oder 2 darstellt, wobei $R_5$ eine $C_1$—$C_6$-Alkylgruppe bedeutet, die durch folgende Reste substituiert ist:

a) durch einen phenylrest, der jeweils auch einen, zwei oder drei gleiche oder verschiedene Substituenten enthalten kann, und wobei die Substituenten Halogenatome, $C_1$—$C_6$-Alkylgruppen oder $C_1$—$C_6$-Alkoxygruppen sind, oder

b) durch einen $C_1$—$C_6$-Alkoxyrest, einen Mono- oder Dihydroxy-$C_1$—$C_6$-alkylrest oder einen Hydroxy-$C_1$—$C_6$-alkoxyrest oder

c) durch einen Mono-$C_1$—$C_6$-alkylamino oder Di-$C_1$—$C_6$-alkylaminorest, wobei letzterer auch zu einem 5—7 gliedrigen stickstoffhaltigen Ring geschlossen sein kann, der gegebenenfalls ein zusätzliches Sauerstoffatom enthält, oder

d) durch einen $C_3$—$C_8$-Cycloalkylrest oder einen $C_3$—$C_8$-Cycloalkylcarbonylrest

oder wobei $R_5$ eine einfach ungesättigte $C_3$—$C_6$-Alkylgruppe, eine einfach ungesättigte $C_3$—$C_6$-Alkylgruppe mit einem Phenylrest, eine einfach ungesättigte $C_3$—$C_6$-Alkylcarbonylgruppe oder eine einfach ungesättigte $C_3$—$C_6$-Alkylcarbonylgruppe mit einem Phenylrest bedeutet

und deren physiologisch unbedenkliche Säureadditionssalze, ausgenommen die Verbindungen, wo

A den Rest

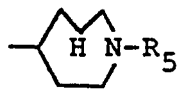

darstellt und $R_5$ Allyl, Benzyl, Phenylethyl oder Methoxyethyl, $R_1$ Chlor in 4-Stellung und $R_2$ und $R_3$ Wasserstoff ist.

2. Verfahren zur Herstellung von 4-Benzyl-1-(2H)-phthalazinon-Derivaten gemäß Anspruch 1, dadurch gekennzeichnet, daß man

a) eine Verbindung der Formel

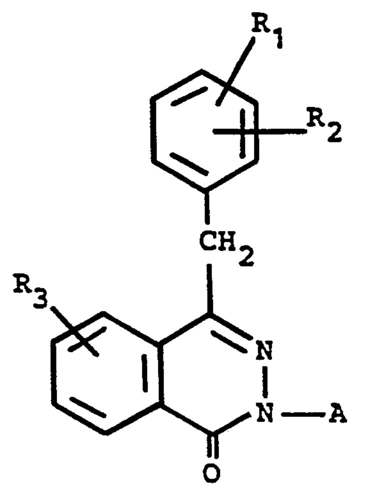

II

worin $R_1$, $R_2$, $R_3$ und A die angegebenen Bedeutungen haben und $R_5$ Wasserstoff ist, mit einer Verbindung der Formel

$$R_5X \qquad III$$

umsetzt, wobei $R_5$ die angegebenen Bedeutungen hat und X eine durch eine starke anorganische oder organische Säure veresterte Hydroxygruppe ist oder

b) eine Verbindung der Formel

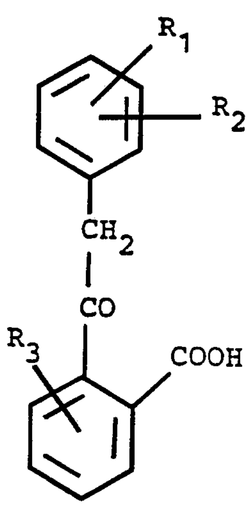

IV

oder ein reaktionsfähiges Derivat derselben worin $R_1$, $R_2$ und $R_3$ die angegebenen Bedeutungen haben, mit einem Hydrazin der allgemeinen Formel

$$H_2N—NH—Z \qquad V$$

worin Z Wasserstoff oder der Rest A ist und A die angegebenen Bedeutungen hat, umsetzt, und in den Fällen, wo Z Wasserstoff ist, die erhaltenen Benzyl-phthalazinon-Derivate anschließend mit einer Verbindung der Formel

$$VI \qquad Y—A$$

umsetzt, worin Y ein Halogenatom oder eine Sulfonsäureestergruppe ist und A die hierfür angegebenen Bedeutungen hat,
gegebenenfalls in die erhaltenen Verbindungen wo $R_1$, $R_2$ Hydroxy-, Amino- oder $C_1$—$C_6$-Alkylaminogruppen darstellen eine $C_1$—$C_6$-Alkylgruppe, und/oder eine $C_2$—$C_6$-Alkanoylgruppe durch Alkylierung oder Acylierung einführt und/oder vorhandene Acylgruppen abspaltet, und gegebenenfalls die erhaltenen Verbindungen in ihre Säureadditionssalze überführt.

3. Verbindungen der Formel I gemäß Anspruch 1 zur Anwendung als therapeutische Wirkstoffe.

4. Arzneimittel, enthaltend eine Verbindung der allgemeinen Formel I gemäß Anspruch 1 neben üblichen Träger- und/oder Verdünnungs- beziehungsweise Hilfsstoffen.

5. Verfahren zur Herstellung eines Arzneimittels, dadurch gekennzeichnet, daß eine Verbindung der allgemeinen Formel I gemäß Anspruch 1 mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

6. Verwendung von Verbindungen der allgemeinen Formel I gemäß Anspruch 1 zur Herstellung von Arzneimitteln.

**Patentansprüche für die Vertragsstaaten: AT ES GR**

1. Verfahren zur Herstellung von 4-Benzyl-1-(2H)-phthalazinon-Derivaten der Formel

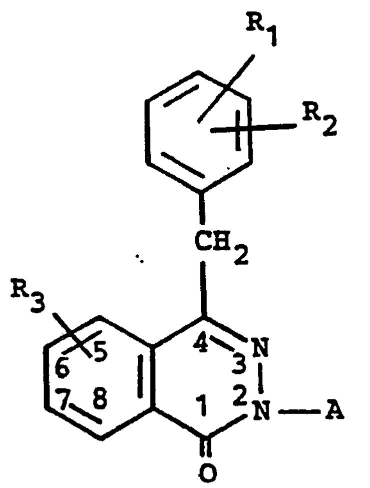

I

worin $R_1$ Fluor, Chlor, Brom, Trifluormethyl, $C_1$—$C_6$-Alkyl, Hydroxy, $C_1$—$C_6$-Alkoxy $C_2$—$C_6$-Alkanoyloxy, Amino, $C_1$—$C_6$-Alkylamino, Di-$C_1$—$C_6$-alkylamino, $C_2$—$C_6$-Alkanoylamino, $C_2$—$C_6$-Alkanoyl-$C_1$—$C_6$-alkylamino oder eine Nitrogruppe bedeutet, $R_2$ Wasserstoff ist oder eine der für $R_1$ angegebenen Bedeutungen hat, wobei $R_1$ und $R_2$ gleich oder verschieden sein können, der Rest $R_3$ Wasserstoff, $C_1$—$C_6$-Alkyl oder Hydroxy bedeutet und

A den Rest 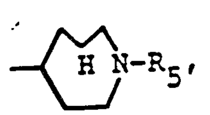

den Rest 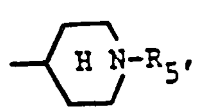

den Rest 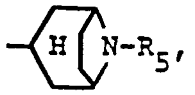

den Rest 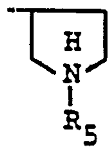 oder

den Rest 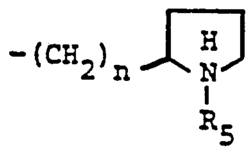

mit n = 1 oder 2 darstellt, wobei $R_5$ eine $C_1$—$C_6$-Alkylgruppe bedeutet, die durch folgende Reste substituiert ist:

a) durch einen Phenylrest, der jeweils auch einen, zwei oder drei gleiche oder verschiedene Substituenten enthalten kann, und wobei die Substituenten Halogenatome, $C_1$—$C_6$-Alkylgruppen oder $C_1$—$C_6$-Alkoxygruppen sind, oder

b) durch einen $C_1$—$C_6$-Alkoxyrest, einen Mono- oder Dihydroxy-$C_1$—$C_6$-alkylrest oder einen Hydroxy-$C_1$—$C_6$-alkoxyrest oder

c) durch einen Mono-$C_1$—$C_6$-alkylamino oder Di-$C_1$—$C_6$-alkylaminorest, wobei letzterer auch zu einem 5—7 gliedrigen stickstoffhaltigen Ring geschlossen sein kann, der gegebenenfalls ein zusätzliches Sauerstoffatom enthält, oder

d) durch einen $C_3$—$C_8$-Cycloalkylrest oder einen $C_3$—$C_8$-Cycloalkylcarbonylrest

oder wobei $R_5$ eine einfach ungesättigte $C_3$—$C_6$-Alkylgruppe, eine einfach ungesättigte $C_3$—$C_6$-Alkylgruppe mit einem Phenylrest, eine einfach ungesättigte $C_3$—$C_6$-Alkylcarbonylgruppe oder eine einfach ungesättigte $C_3$—$C_6$-Alkylcarbonylgruppe mit einem Phenylrest bedeutet

und deren physiologisch unbedenklichen Säureadditionssalzen, ausgenommen die Verbindungen, wo

A den Rest 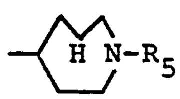

darstellt und $R_5$ Allyl, Benzyl, Phenylethyl oder Methoxyethyl, $R_1$ Chlor in 4-Stellung und $R_2$ und $R_3$ Wasserstoff ist, dadurch gekennzeichnet, daß man

a) eine Verbindung der Formel

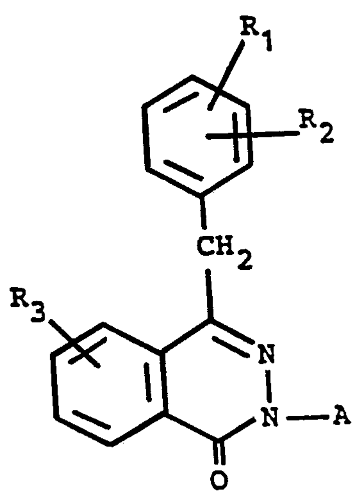

II

worin $R_1$, $R_2$, $R_3$ und A die angegebenen Bedeutungen haben und $R_5$ Wasserstoff ist, mit einer Verbindung der Formel

$R_5X$ III

umsetzt, wobei $R_5$ die angegebenen Bedeutungen hat und X eine durch eine starke anorganische oder organische Säure veresterte Hydroxygruppe ist oder

b) eine Verbindung der Formel

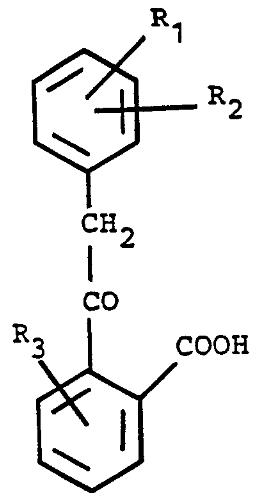

IV

oder ein reaktionsfähiges Derivat derselben worin $R_1$, $R_2$ und $R_3$ die angegebenen Bedeutungen haben, mit einem Hydrazin der allgemeinen Formel

$$H_2N—NH—Z \qquad V$$

worin Z Wasserstoff oder der Rest A ist und A die angegebenen Bedeutungen hat, umsetzt, und in den Fällen, wo Z Wasserstoff ist, die erhaltenen Benzyl-phthalazinon-Derivate anschließend mit einer Verbindung der Formel

$$Y—A \qquad VI$$

umsetzt, worin Y ein Halogenatom oder eine Sulfonsäureestergruppe ist und A die hierfür angegebenen Bedeutungen hat,

gegebenenfalls in die erhaltenen Verbindungen wo $R_1$, $R_2$ Hydroxy-, Amino- oder $C_1$—$C_6$-Alkylaminogruppen darstellen eine $C_1$—$C_6$-Alkylgruppe, und/oder eine $C_2$—$C_6$-Alkanoylgruppe durch Alkylierung oder Acylierung einführt und/oder vorhandene Acylgruppen abspaltet, und gegebenenfalls die erhaltenen Verbindungen in ihre Säureadditionssalze überführt.

2. Verfahren zur Herstellung eines Arzneimittels, enthaltend als Wirkstoff mindestens eine Verbindung der allgemeinen Formel I, worin die Reste $R_1$, $R_2$, $R_3$, $R_5$ und A die in Anspruch 1 angegebene Bedeutung haben, dadurch gekennzeichnet, daß mindestens eine Verbindung der Formel I mit gebräuchlichen pharnazeutischen Trägerstoffen und/oder Verdünnungsmitteln beziehungsweise sonstigen Hilfsstoffen zu pharmazeutischen Zubereitungen verabeitet beziehungsweise ub eine therapeutisch anwendbare form gebracht wird.

**Revendications pour les Etats contractants: BE CH DE FR GB IT LI LU NL SE**

1. Dérivés de 4-benzyl-1-(2H)-phtalazinone de formule

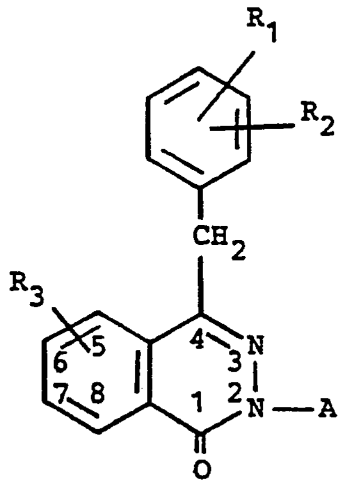

I

dans laquelle $R_1$ représente un atome de fluor, de chlore, de brome ou un groupement trifluorométhyle, alkyle en $C_1$—$C_6$, hydroxy, alcoxy en $C_1$—$C_6$, alcanoyloxy en $C_1$—$C_6$, amino, $C_1$—$C_6$-alkylamino, di-$C_1$—$C_6$-alkylamino, $C_2$—$C_6$-alcanoylamino, $C_2$—$C_6$-alcanoyl-$C_1$—$C_6$-alkylamino ou nitro, $R_2$ est un atome d'hydrogène ou a l'une des significations données pour $R_1$, $R_1$ et $R_2$ pouvant être identiques ou différents, le reste $R_3$ est un atome d'hydrogène ou un groupement alkyle en $C_1$—$C_6$ ou hydroxy, et A représente

le reste 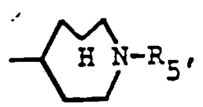

le reste 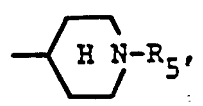

le reste 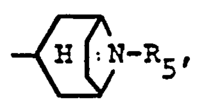

le reste 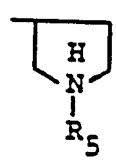 ou

le reste 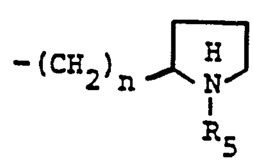

avec n = 1 ou 2, $R_5$ représentant un groupement alkyle en $C_1$—$C_6$ qui est substitué par les restes suivants:

a) par un reste phényle qui peut renfermer à son tour un, deux ou trois substituants identiques ou différents, les substituants étant des atomes d'halogènes, des groupements alkyle en $C_1$—$C_6$ ou des groupements alcoxy en $C_1$—$C_6$,

b) par un reste alcoxy en $C_1$—$C_6$, un reste mono- ou dihydroxy-$C_1$—$C_6$-alkyle ou un reste hydroxy-$C_1$—$C_6$-alcoxy,

c) par un reste mono-$C_1$—$C_6$-alkylamino ou di-$C_1$—$C_6$-alkylamino, ce dernier pouvant être également fermé en un noyau penta- à heptagonal azoté qui contient éventuellement un atome d'oxygène supplémentaire, ou

d) par un reste cycloalkyle en $C_3$—$C_8$ ou un reste $C_3$—$C_8$-cycloalkylcarbonyle,

ou bien $R_5$ représentant un groupement alkyle en $C_3$—$C_6$ à simple insaturation, un groupement en $C_3$—$C_6$ à simple insaturation avec un reste phényle, un groupement $C_3$—$C_6$-alkylcarbonyle à simple insaturation ou un groupement $C_3$—$C_6$-alkylcarbonyle à simple insaturation avec un reste phényle,

et leurs sels formés par addition d'acides physiologiquement inoffensifs,

exception faite des composés où A représente le reste

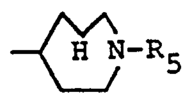

et $R_5$ est un reste allyle, benzyle, phényléthyle ou méthoxyéthyle, $R_1$ est un atome de chlore en position 4 et $R_2$ et $R_3$ sont des atomes d'hydrogène.

2. Procédé de préparation de dérivés de 4-benzyl-1-(2H)-phtalazinone selon la revendication 1, caractérisé en ce qu'on fait réagir

a) un composé de formule

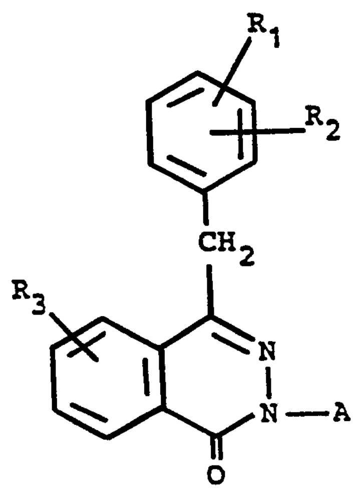

II

dans laquelle $R_1$, $R_2$, $R_3$ et A ont les significations indiquées et $R_5$ est un atome d'hydrogène, avec un composé de formule

$$R_5X \qquad III$$

$R_5$ ayant les significations indiquées et X étant un groupement hydroxy estérifié par un acide organique ou minéral fort,

ou

b) on fait réagir un composé de formule

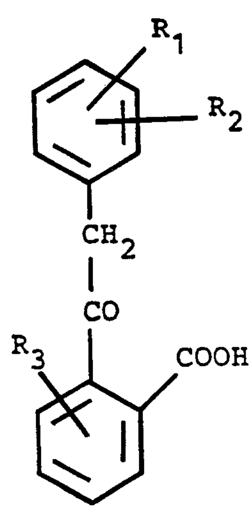

ou un dérivé réactif de celui-ci, $R_1$, $R_2$ et $R_3$ ayant les significations indiquées, avec une hydrazine de formule générale

$$H_2N—NH—Z \qquad V$$

dans laquelle Z est un atome d'hydrogène ou le reste A et A a les significations indiquées, et, dans les cas où Z est un atome d'hydrogène, on fait ensuite réagir les dérivés de benzyl-phtalazinone obtenus avec un composé de formule

$$Y—A \qquad VI$$

dans laquelle Y est un atome d'halogène ou un groupement ester d'acide sulfonique et A a les significations indiquées,

le cas échéant, dans les composés obtenus où $R_1$, $R_2$ représentent des groupements hydroxy, amino ou $C_1$—$C_6$-alkylamino, on introduit un groupement alkyle en $C_1$—$C_6$ et/ou un groupement alcanoyle en $C_2$—$C_6$ par alkylation ou acylation et/ou on élimine des groupements acyle présents, et on transforme éventuellement les composés obtenus en leurs sels formés par addition d'acide.

3. Composés de formule I selon la revendication 1, destinés à être utilisés comme substances actives thérapeutiques.

4. Médicament, contenant un composé de formule générale I selon la revendication 1, outre des excipients et/ou diluants ou adjuvants usuels.

5. Procédé de préparation d'un médicament, caractérisé en ce qu'on transforme en préparations pharmaceutiques un composé de formule générale I selon la revendication 1 avec des excipients et/ou des diluants pharmaceutiques usuels ou avec d'autres adjuvants, ou on le met sous une forme se prêtant à l'application thérapeutique.

6. Utilisation de composé de formule générale I selon la revendication 1 pour la préparation de médicaments.

**Revendications pour les Etats contractants: AT ES GR**

1. Procédé de préparation de dérivés de 4-benzyl-1-(2H)-phtalazinone de formule

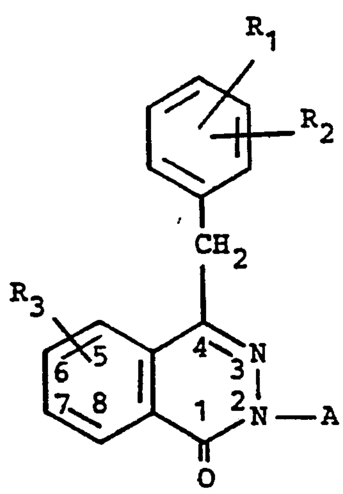

I

dans laquelle $R_1$ représente un atome de fluor, de chlore, de brome ou un groupement trifluorométhyle, alkyle en $C_1$—$C_6$, hydroxy, alcoxy en $C_1$—$C_6$, alcanoyloxy en $C_1$—$C_6$, amino, $C_1$—$C_6$-alkylamino, di-$C_1$—$C_6$-alkylamino, $C_2$—$C_6$-alcanoylamino, $C_2$—$C_6$-alcanoyl-$C_1$—$C_6$-alkylamino ou nitro, $R_2$ est un atome d'hydrogène ou a l'une des significations données pour $R_1$, $R_1$ et $R_2$ pouvant être identiques ou différents, le reste $R_3$ est un atome d'hydrogène ou un groupement alkyle en $C_1$—$C_6$ ou hydroxy, et A représente

le reste 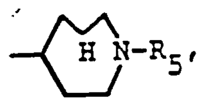

le reste 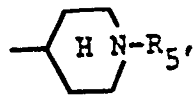

le reste 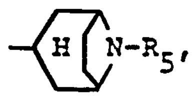

le reste 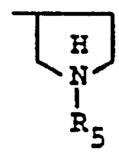 ou

le reste 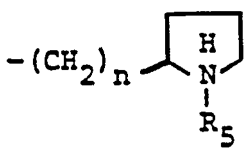

avec n = 1 ou 2, $R_5$ représentant un groupement alkyle en $C_1$—$C_6$ qui est substitué par les restes suivants:

a) par un reste phényle qui peut renfermer à son tour un, deux ou trois substituants identiques ou différents, les substituants étant des atomes d'halogènes, des groupements alkyle en $C_1$—$C_6$ ou des groupements alcoxy en $C_1$—$C_6$,

b) par un reste alcoxy en $C_1$—$C_6$, un reste mono- ou dihydroxy-$C_1$—$C_6$-alkyle ou un reste hydroxy-$C_1$—$C_6$-alcoxy,

c) par un reste mono-$C_1$—$C_6$-alkylamino ou di-$C_1$—$C_6$-alkylamino, ce dernier pouvant être également fermé en un noyau penta- à heptagonal azoté qui contient éventuellement un atome d'oxygène supplémentaire, ou

d) par un reste cycloalkyle en $C_3$—$C_8$ ou un reste $C_3$—$C_8$-cycloalkylcarbonyle,

ou bien $R_5$ représentant un groupement alkyle en $C_3$—$C_6$ à simple insaturation, un groupement en $C_3$—$C_6$ à simple insaturation avec un reste phényle, un groupement $C_3$—$C_6$-alkylcarbonyle à simple insaturation ou un groupement $C_3$—$C_6$-alkylcarbonyle à simple insaturation avec un reste phényle,

et de leurs sels formés par addition d'acides physiologiquement inoffensifs,

exception faite des composés où A représente le reste

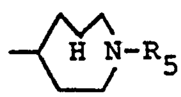

et $R_5$ est un reste allyle, benzyle, phényléthyle ou méthoxyéthyle, $R_1$ est un atome de chlore en position 4 et $R_2$ et $R_3$ sont des atomes d'hydrogène, caractérisé en ce qu'on fait réagir

a) un composé de formule

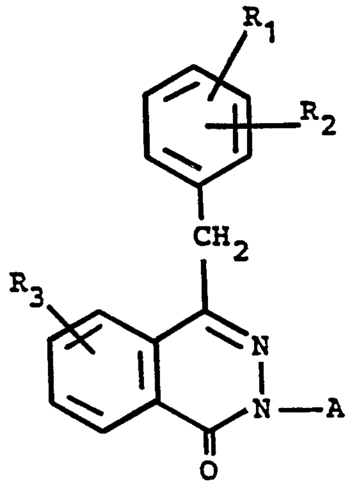

II

dans laquelle $R_1$, $R_2$, $R_3$ et A ont les significations indiquées et $R_5$ est un atome d'hydrogène, avec un composé de formule

$$R_5X \qquad III$$

$R_5$ ayant les significations indiquées et X étant un groupement hydroxy estérifié par un acide organique ou minéral fort,

ou

b) on fait réagir un composé de formule

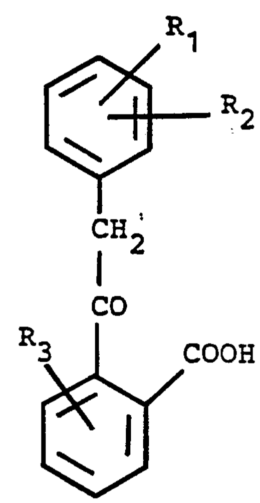

IV

ou un dérivé réactif de celui-ci, $R_1$, $R_2$ et $R_3$ ayant les significations indiquées, avec une hydrazine de formule générale

$$H_2N—NH—Z \qquad V$$

dans laquelle Z est un atome d'hydrogène ou le reste A et A a les significations indiquées, et, dans les cas où Z est un atome d'hydrogène, on fait ensuite réagir les dérivés de benzyl-phtalazinone obtenus avec un composé de formule

$$Y—A \qquad VI$$

dans laquelle Y est un atome d'halogène ou un groupement ester d'acide sulfonique et A a les significations indiquées,

le cas échéant, dans les composés obtenus où $R_1$, $R_2$ représentent des groupements hydroxy, amino ou $C_1$—$C_6$-alkylamino, on introduit un groupement alkyle en $C_1$—$C_6$ et/ou un groupement alcanoyle en $C_2$—$C_6$ par alkylation ou acylation et/ou on élimine des groupements acyle présents, et on transforme éventuellement les composés obtenus en leurs sels formés par addition d'acide.

2. Procédé de préparation d'un médicament contenant, en tant que substance active, au moins un composé de formule générale I dans laquelle le restes $R_1$, $R_2$, $R_3$, $R_5$ et A ont les significations données dans la revendication 1, caractérisé en ce qu'au moins un composé de formule I est transformé en préparations pharmaceutiques avec des excipients et/ou des diluants pharmaceutiques usuels ou avec d'autres adjuvants, ou est mis sous une forme se prêtant à l'application thérapeutique.

**Claims for the Contracting States: BE CH DE FR GB IT LI LU NL SE**

1. 4-Benzyl-1-(2H)-phthalazinone derivatives corresponding to the following formula

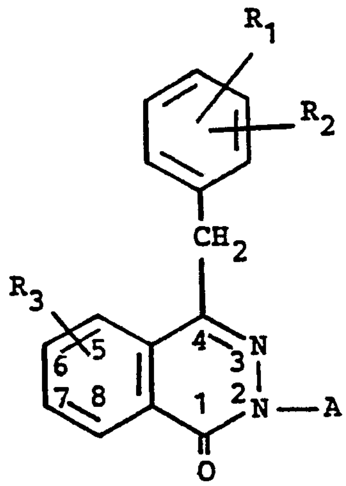

I

in which $R_1$ represents fluorine, chlorine, bromine, trifluoromethyl, $C_{1-6}$-alkyl, hydroxy, $C_{1-6}$-alkoxy, $C_{2-6}$ alkanoyloxy, amino, $C_{1-6}$-alkylamino, di-$C_{1-6}$-alkylamino, $C_{2-6}$-alkanoylamino, $C_{2-6}$-alkanoyl-$C_{1-6}$-alkylamino or a nitro group, $R_2$ is hydrogen or has any one of the meanings defined for $R_1$, $R_1$ and $R_2$ being the same of different; $R_3$ represents hydrogen, $C_{1-6}$ alkyl or hydroxy and A represents

the group 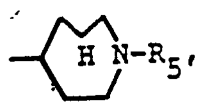

the group 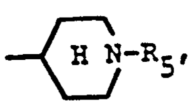

the group 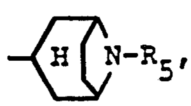

the group 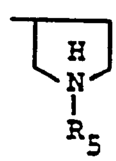 or

the group 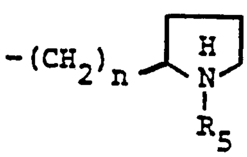

where n = 1 or 2 and $R_5$ is a $C_{1-6}$-alkyl group substituted by the following radicals:

a) by a phenyl radical which may also contain one, two or three identical or different substituents, the substituents in question being halogen atoms, $C_{1-6}$-alkyl groups or $C_{1-6}$-alkoxy groups, or

b) by a $C_{1-6}$-alkoxy radical, a monoor dihydroxy-$C_{1-6}$-alkyl radical or a hydroxy-$C_{1-6}$-alkoxy radical or

c) by a mono-$C_{1-6}$-alkylamino or di-$_{1-6}$-alkylamino radical which may even be closed to form a 5 to 7-membered nitrogen-containing ring optionally containing an additional oxygen atom or

d) by a $C_{3-8}$ cycloalkyl radical or a $C_{3-8}$ cycloalkylcarbonyl radical

or $R_5$ is a monounsaturated $C_{3-6}$-alkyl group, a monounsaturated $C_{3-6}$-alkyl group containing a phenyl radical, a monounsaturated $C_{3-6}$-alkylcarbonyl group or a monounsaturated $C_{3-6}$-alkylcarbonyl group containing a phenyl radical, and physiologically acceptable acid addition salts thereof, except for those compounds in which A is the group

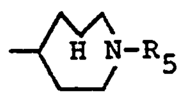

and $R_5$ is allyl, benzyl, phenylethyl or methoxyethyl, $R_1$ is chlorine in the 4-position and $R_2$ and $R_3$ are hydrogen.

2. A process for the production of the 4-benzyl-1-(2H)-phthalazinone derivatives claimed in claim 1, characterized in that

a) a compound corresponding to the following formula

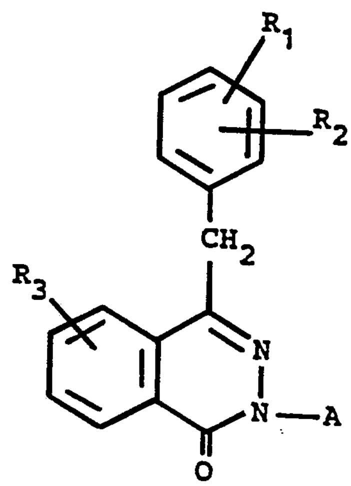

II

in which $R_1$, $R_2$, $R_3$ and A are as defined above and $R_5$ is hydrogen, is reacted with a compound corresponding to the following formula

$R_5X$ III

in which $R_5$ is as defined above and X is a hydroxy group esterified by a strong inorganic or organic acid, or

b) a compound corresponding to the following formula

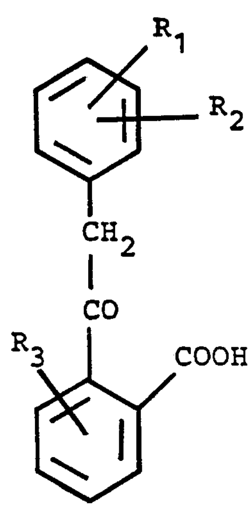

IV

in which $R_1$, $R_2$ and $R_3$ are as defined above, or a reactive derivative thereof is reacted with a hydrazine corresponding to the following general formula

$$H_2N—NH—Z \qquad V$$

in which Z is hydrogen or the group A and A is as defined above and, in cases where Z is hydrogen, the benzyl phthalazinone derivatives obtained are subsequently reacted with a compound corresponding to the following formula

$$Y—A \qquad VI$$

in which Y is a halogen atom or a sulfonic acid ester group and A is as defined above, and a $C_{1-6}$ alkyl group and/or a $C_{2-6}$-alkanoyl group is optionally introduced into the resulting compounds, in which $R_2$ represents hydroxy, amino or $C_{1-6}$ alkylamino groups, by alkylation or acylation and/or any acyl groups present are eliminated and the compounds obtained are optionally converted into their acid addition salts.

3. Compounds corresponding to formula I in claim 1 for use as therapeutic agents.

4. Medicaments containing a compound corresponding to general formla I in claim 1 in addition to typical excipients and/or diluents and auxiliaries.

5. A process for the preparation of a medicament, characterized in that a compound corresponding to general formula I in claim 1 is processed to pharmaceutical preparations or brought into a therapeutically useable form with standard pharmaceutical excipients and/or diluents and other auxiliaries.

6. The use of compounds corresponding to general formula I in claim 1 for the production of medicaments.

**Claims for the Contracting States: AT ES GR**

1. A process for the production of 4-benzyl-1-(2H)-phthalazinone derivatives corresponding to the following formula

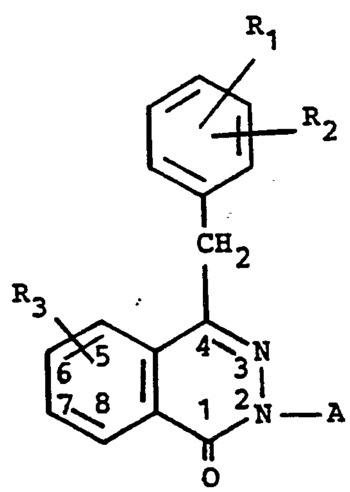

I

in which $R_1$ represents fluorine, chlorine, bromine, trifluoromethyl, $C_{1-6}$ alkyl, hydroxy, $C_{1-6}$ alkoxy, $C_{2-6}$ alkanoyloxy, amino, $C_{1-6}$-alkylamino, di-$C_{1-6}$-alkylamino, $C_{2-6}$-alkanoylamino, $C_{2-6}$-alkanoyl-$C_{1-6}$-alkylamino or a nitro group, $R_2$ is hydrogen or has any one of the meanings defined for $R_1$, $R_1$ and $R_2$ being the same or different; $R_3$ represents hydrogen, $C_{1-6}$ alkyl or hydroxy and A represents

the group 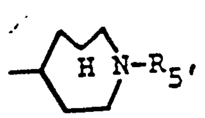

the group 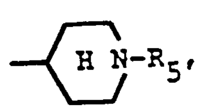

the group 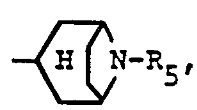

the group

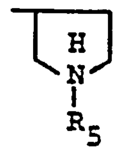

or

the group

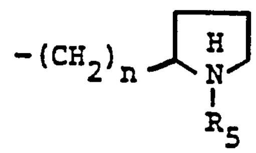

where n = 1 or 2 and $R_5$isa $C_{1-6}$ alkyl group substituted by the following radicals:

a) by a phenyl radical which may also contain one, two or three identical or different substituents, the substituents in question being halogen atoms, $C_{1-6}$-alkyl groups or $C_{1-6}$-alkoxy groups, or

b) by a $C_{1-6}$ alkoxy radical, a monoor dihydroxy-$C_{1-6}$-alkyl radical or a hydroxy-$C_{1-6}$-alkoxy radical or

c) by a mono-$C_{1-6}$-alkylamino or di-$_{1-6}$-alkylamino radical which may even be closed to form a 5 to 7-membered nitrogen-containing ring optionally containing an additional oxygen atom or

d) by a $C_{3-8}$ cycloalkyl radical or a $C_{3-8}$ cycloalkylcarbonyl radical

or $R_5$ is a monounsaturated $C_{3-6}$ alkyl group, a monounsaturated $C_{3-6}$ alkyl group containing a phenyl radical, a monounsaturated $C_{3-6}$ alkylcarbonyl group or a monounsaturated $C_{3-6}$ alkylcarbonyl group containing a phenyl radical, and physiologically acceptable acid addition salts thereof,

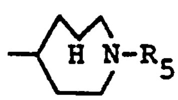

except for those compounds in which A is the group and $R_5$ is allyl, benzyl, phenylethyl or methoxyethyl, $R_1$ is chlorine in the 4-position and $R_2$ and $R_3$ are hydrogen, characterized in that

a) a compound corresponding to the following formula

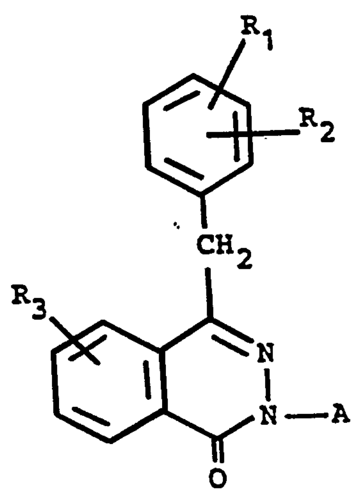

II

in which $R_1$, $R_2$, $R_3$ and A are as defined above and $R_5$ is hydrogen, is reacted with a compound corresponding to the following formula

$$R_5X \qquad III$$

in which $R_5$ is as defined above and X is a hydroxy group esterified by a strong inorganic or organic acid, or

b) a compound corresponding to the following formula

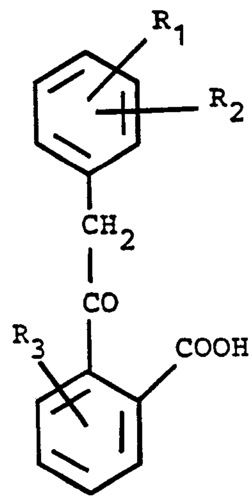

IV

in which $R_1$, $R_2$ and $R_3$ are as defined above, or a reactive derivative thereof is reacted with a hydrazine corresponding to the following general formula

$$H_2N—NH—Z \qquad V$$

in which Z is hydrogen or the group A and A is as defined above and, in cases where Z is hydrogen, the benzyl phthalazinone derivatives obtained are subsequently reacted with a compound corresponding to the following formula

$$Y—A \qquad (VI)$$

in which Y is a halogen atom or a sulfonic acid ester group and A is as defined above, and a $C_{1-6}$ alkyl group and/or a $C_{2-6}$ alkanoyl group is optionally introduced into the resulting compounds, in which $R_2$ represents hydroxy, amino or $C_{1-6}$ alkylamino groups, by alkylation or acylation and/or any acyl groups present are eliminated and the compounds obtained are optionally converted into their acid addition salts.

2. A process for the production of a medicament containing as active principle at least one compound corresponding to general formula I, in which $R_1$, $R_2$, $R_3$, $R_5$ and A are as defined in claim 1, characterized in that at least one compound corresponding to general formula I is processed to pharmaceutical preparations or brought into a therapeutically useable form with standard pharmaceutical excipients and/or diluents and other auxiliaries.